# EUROPEAN PATENT APPLICATION

(11) **EP 3 766 990 A1**
(43) Date of publication of application: **20.01.2021**
(21) Application number: 19186396.8
(22) Date of filing: 15.07.2019
(51) Int. Cl.: C12Q 1/6827, C12Q 1/6858

(54) **PRIMERS FOR ISOTHERMAL AMPLIFICATION**

(71) Applicant: National University of Ireland Galway, Galway (IE)
(72) Inventor: Higgins, Owen, Galway (IE); Smith, Terry, Galway (IE)
(74) Representative: FRKelly

(57) **Abstract**

The present invention relates to primers for isothermal amplification, in particular primers for loop-mediated isothermal amplification (LAMP). Also disclosed are methods for identifying a target nucleic acid, and methods for identifying a nucleic acid modification or substitution of a target nucleic acid. The invention finds utility in the diagnosis of diseases or disorders.

## Description

### Field of the Invention

The present invention relates to primers for isothermal amplification, in particular primers for loop-mediated isothermal amplification (LAMP), methods for performing LAMP, and methods for diagnosing a disease or disorder using LAMP.

### Background to the Invention

Nucleic acid amplification technologies provide improved alternatives to conventional culture-based methods for the diagnosis of infectious diseases in terms of diagnostic sensitivity and specificity, time-to-detection, reduced contamination and high throughput capabilities. Real-time polymerase chain reaction (PCR) is the benchmark nucleic acid diagnostic technology. However, for low-resourced, disease-burdened regions it is an impractical point-of-care (POC) diagnostic option due to requirement of expensive thermocycling equipment. Isothermal nucleic acid amplification techniques do not require thermocycling and thus offer a more convenient diagnostic option, and loop-mediated isothermal amplification (LAMP) is one of the most commonly used single-temperature nucleic acid amplification methods.

LAMP incorporates strand-displacing bacterial DNA polymerase with target-specific forward and reverse outer primers, forward and reverse inner primers, and optionally forward and reverse loop primers. Typical LAMP reactions are performed at a single temperature ranging from 60°C to 65°C, enabling initial target hybridisation by the inner and outer primers. Strand-displacing primer extension combined with the sense and antisense inner primer sequences facilities the loop structure formation in LAMP, producing a unique double-looped DNA template. This template is targeted by the inner and loop primers, leading to rapid exponential target amplification. LAMP possesses single-digit genome copy sensitivity and very high specificity as the 4-6 target-specific primers recognise 6-8 distinct regions on the target nucleic acid. Monitoring LAMP reactions can be performed using direct end-point visualisation or real-time turbidimetric analysis of magnesium pyrophosphate precipitation, a polymerisation by-product. Alternative real-time monitoring or post-amplification analysis of LAMP reactions can be achieved using intercalating, colourimetric, or pH sensitive dyes. LAMP is user-friendly, cost-effective, robust, capable of amplifying nucleic acid from samples without prior extraction and compatible with basic POC detection technologies, making it an ideal near-patient diagnostic option.

However, the non-exonuclease strand-displacement activity of the commonly used bacterial DNA polymerase in LAMP is not compatible with standard nucleic acid hybridisation probes, making multiplex detection very difficult. Clinical application of nucleic acid diagnostics requires multiplex detection capabilities for simultaneous pathogen detection, reduced analysis time, conservation of sample and incorporation of assay validating internal controls.

An SNP is a single nucleotide sequence variation at a specific genome location, present in at least 1% of a population. SNPs are the simplest and most abundant form of genetic sequence variation occurring approximately once in every 1,000 bases. Typically, SNPs are biallelic (two allele variants), with tri-allelic or tetra-allelic variants presenting less frequently, and are predominantly located in non-coding genome regions with minimal phenotypic impact. SNPs located in genome coding regions contribute to phenotypic variations, disease development and altered responses to drugs or environmental toxins. Various SNPs are associated with cancer, cardiovascular disorders, diabetes, autoimmune diseases, gastrointestinal disorders and infectious diseases. As a result, SNPs are commonly utilised as biomarkers for gene mapping and disease association studies, development of personalised medicines in pharmacogenetics, and molecular diagnostics. DNA sequencing is widely used for SNP analysis, however, due to the requirement of extensive instrumentation and data analysis, this approach is more practical for SNP discovery instead of rapid POC application. Nucleic acid infectious disease diagnostics utilise SNPs, and pathogen point-mutations associated with antimicrobial resistance, for accurate disease diagnosis facilitating improved treatment and reduced antimicrobial resistance dissemination. Nucleic acid diagnostic methods with SNP genotyping capabilities also enable greater specificity with effective differentiation of closely related pathogens. Typical nucleic acid SNP genotyping approaches involve differentiation of wild-type and mutant alleles using either allele-specific hybridisation or allele-specific enzymatic methods.

Combining SNP detection capabilities with rapid, sensitive and specific, multiplex isothermal nucleic acid techniques, such as LAMP, would provide very effective and transferable diagnostics technology for POC application.

### Summary of the Invention

According to a first aspect of the present invention there is provided a set of primers for isothermal amplification comprising (a) a forward outer primer; (b) a reverse outer primer; (c) a forward inner primer; and (d) a reverse inner primer;
wherein each primer is capable of binding to a target nucleic acid having complementary first and second nucleic acid strands,
wherein the first nucleic acid strand has first, second, third, fourth, fifth and sixth regions; and
wherein the second nucleic acid strand has first, second, third, fourth, fifth and sixth regions;
wherein the fourth, fifth and sixth regions of the first nucleic acid strand are complimentary to the third, second, and first regions of the second nucleic acid strand; and
wherein the fourth, fifth and sixth regions of the second nucleic acid strand are complimentary to the third, second, and first regions of the first nucleic acid strand; and
wherein
(a) the forward outer primer is complementary to the first region of the first nucleic acid strand;
(b) the reverse outer primer is complementary to the first region of the second nucleic acid strand;
(c) the forward inner primer has first and second parts, wherein:
   (i) the first part is complementary to the second region of the first nucleic acid strand; and
   (ii) the second part is complementary to the fourth region of the second nucleic acid strand; and
(d) the reverse inner primer has first and second parts, wherein:
   (i) the first part is complementary to the second region of the second nucleic acid strand; and
   (ii) the second part is complementary to the fourth region of the first nucleic acid strand;
wherein at least one of the (c) forward inner and (d) reverse inner primers comprises a reporter.

Optionally, the set of primers further comprises (e) a forward loop primer and/or (f) a reverse loop primer.

Optionally, the set of primers comprises (a) a forward outer primer; (b) a reverse outer primer; (c) a forward inner primer; (d) a reverse inner primer, and (e) a forward loop primer and/or (f) a reverse loop primer;
wherein each primer is capable of binding to a target nucleic acid having complementary first and second nucleic acid strands,
wherein the first nucleic acid strand has first, second, third, fourth, fifth and sixth regions; and
wherein the second nucleic acid strand has first, second, third, fourth, fifth and sixth regions;
wherein the fourth, fifth and sixth regions of the first nucleic acid strand are complimentary to the third, second, and first regions of the second nucleic acid strand; and
wherein the fourth, fifth and sixth regions of the second nucleic acid strand are complimentary to the third, second, and first regions of the first nucleic acid strand; and
wherein
(a) the forward outer primer is complementary to the first region of the first nucleic acid strand;
(b) the reverse outer primer is complementary to the first region of the second nucleic acid strand;
(c) the forward inner primer has first and second parts, wherein:
   (i) the first part is complementary to the second region of the first nucleic acid strand; and
   (ii) the second part is complementary to the fourth region of the second nucleic acid strand; and
(d) the reverse inner primer has first and second parts, wherein:
   (i) the first part is complementary to the second region of the second nucleic acid strand; and
   (ii) the second part is complementary to the fourth region of the first nucleic acid strand;
(e) the forward loop primer is complementary to a region between the fourth and fifth regions of the second nucleic acid strand;
(f) the reverse loop primer is complementary to a region between the fourth and fifth regions of the first nucleic acid strand;
wherein at least one of the (c) forward inner, (d) reverse inner, (e) forward loop, and (f) reverse loop primers comprises the reporter.

Optionally, the set of primers comprises (a) a forward outer primer; (b) a reverse outer primer; (c) a forward inner primer; (d) a reverse inner primer, and (e) a forward loop primer.

Optionally, the set of primers comprises (a) a forward outer primer; (b) a reverse outer primer; (c) a forward inner primer; (d) a reverse inner primer, and (f) a reverse loop primer.

Optionally, the set of primers comprises (a) a forward outer primer; (b) a reverse outer primer; (c) a forward inner primer; (d) a reverse inner primer, (e) a forward loop primer and (f) a reverse loop primer.

Optionally, the (c) forward inner primer comprises the reporter.

Optionally, the (d) reverse inner primer comprises the reporter.

Optionally, the (e) forward loop primer comprises the reporter.

Optionally, the (f) reverse loop primer comprises the reporter.

Optionally, the set of primers comprises (a) a forward outer primer; (b) a reverse outer primer; (c) a forward inner primer; (d) a reverse inner primer, (e) a forward loop primer and (f) a reverse loop primer; and the (e) forward loop primer comprises the reporter.

Optionally, the set of primers comprises (a) a forward outer primer; (b) a reverse outer primer; (c) a forward inner primer; (d) a reverse inner primer, (e) a forward loop primer and (f) a reverse loop primer; and the (f) reverse loop primer comprises the reporter.

Optionally, the set of primers comprises (a) a forward outer primer; (b) a reverse outer primer; (c) a forward inner primer; (d) a reverse inner primer, (e) a forward loop primer and (f) a reverse loop primer; and the (e) forward loop primer and the (f) reverse loop primer each comprise the reporter.

According to a second aspect of the present invention, there is provided a method for identifying a target nucleic acid, the method comprising:
(a) providing a sample;
(b) providing a set of primers according to the first aspect of the present invention;
(c) performing isothermal amplification; and
(d) identifying the nucleic acid target in the sample.

According to a third aspect of the present invention, there is provided a method for identifying a nucleic acid modification or substitution of a target nucleic acid, the method comprising:
(a) providing a sample;
(b) providing a set of primers according to the first aspect of the present invention;
(c) performing isothermal amplification; and
(d) identifying the nucleic acid modification or substitution of the target nucleic acid in the sample.

According to a fourth aspect of the present invention, there is provided a method for diagnosing a disease or disorder, the method comprising:
(a) providing a sample;
(b) providing a set of primers according to the first aspect of the present invention;
(c) performing isothermal amplification; and
(d) diagnosing the disease or disorder.

Optionally, the isothermal amplification is loop-mediated isothermal amplification (LAMP).

Optionally, the reporter is attached to at least one of the (c) forward inner primer, (d) reverse inner primer, (e) forward loop primer, and (f) reverse loop primer.

Optionally, the reporter is reversibly attached to at least one of the (c) forward inner primer, (d) reverse inner primer, (e) forward loop primer, and (f) reverse loop primer.

Optionally, the reporter is attached at or adjacent a terminal end of at least one of the (c) forward inner primer, (d) reverse inner primer, (e) forward loop primer, and (f) reverse loop primer.

Optionally, the reporter is attached at or adjacent the 5' terminal end of at least one of the (c) forward inner primer, (d) reverse inner primer, (e) forward loop primer, and (f) reverse loop primer.

Optionally, at least one of the (c) forward inner primer, (d) reverse inner primer, (e) forward loop primer, and (f) reverse loop primer further comprises a cleavage site.

Optionally, at least one of the (c) forward inner primer, (d) reverse inner primer, (e) forward loop primer, and (f) reverse loop primer further comprises an endonuclease cleavage site.

Optionally, at least one of the (c) forward inner primer, (d) reverse inner primer, (e) forward loop primer, and (f) reverse loop primer further comprises a cleavage site at or adjacent the reporter.

Optionally, at least one of the (c) forward inner primer, (d) reverse inner primer, (e) forward loop primer, and (f) reverse loop primer further comprises an endonuclease cleavage site at or adjacent the reporter.

Optionally, the reporter is attached at or adjacent the 5' terminal end of the cleavage site.

Optionally, the cleavage site is an apurinic/apyrimidinic site (abasic site).

Optionally, the reporter emits a signal.

Optionally, the reporter emits a detectable signal.

Optionally, the signal is an electrochemical or electromagnetic signal. Further optionally, the signal is an electromagnetic signal.

Optionally, the signal is a light signal. Further optionally, the signal is a visible light signal.

Optionally, the signal is a colorimetric, luminescent, fluorescent, or phosphorescent signal. Further optionally, the signal is a fluorescent signal.

Optionally, the reporter comprises at least one dye.

Optionally, the reporter comprises first and second dyes.

Optionally, the first and second dyes are not the same dye.

Optionally, at least one of the (c) forward inner primer, (d) reverse inner primer, (e) forward loop primer, and (f) reverse loop primer comprises first and second dyes, and a cleavage site.

Optionally, the first and second dyes are located at or adjacent opposing ends of the cleavage site.

Optionally, the cleavage site is located between the first and second dyes.

Optionally, the reporter comprises at least one dye and at least one quencher.

Optionally, at least one of the (c) forward inner primer, (d) reverse inner primer, (e) forward loop primer, and (f) reverse loop primer comprises at least one dye and at least one quencher, and a cleavage site.

Optionally, the at least one dye and at least one quencher are located at or adjacent opposing ends of the cleavage site.

Optionally, the cleavage site is located between the at least one dye and at least one quencher.

Optionally, the at least one dye is a chromophore, or fluorophore. Further optionally, the dye is a fluorophore.

Optionally, the at least one dye is selected from a derivative of any of: xanthene; cyanine; squaraine; squaraine rotaxane; naphthalene; coumarin; oxadiazole; anthracene; pyrene; oxazine; acridine; arylmethine; and tetrapyrrole.

Optionally, the at least one dye is selected from any of: fluorescein, rhodamine, Oregon green, eosin, Texas red, cyanine, indocarbocyanine, oxacarbocyanine, thiacarbocyanine, merocyanine, dansyl, prodan, pyridyloxazole, nitrobenzoxadiazole, benzoxadiazole, anthraquinones, cascade blue, Nile red, Nile blue, cresyl violet, oxazine 170, proflavin, acridine orange, acridine yellow auramine, crystal violet, malachite green, porphin, phthalocyanine, and bilirubin.

Optionally, the at least one dye is selected from 3',6'-dihydroxy-1-oxospiro[2-benzofuran-3,9'-xanthene]-5-carboxylic acid (6-Carboxyfluorescein; 6-FAM); 6-Carboxy-2',7'-dichlorofluorescein diacetate N-succinimidyl ester (6-hexachlorofluorescein; 6-HEX); and 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5).

Optionally, the quencher is selected from molecular oxygen, iodide ions, chloride ions, and acrylamide.

Optionally, the quencher is a non-fluorescent chromophore.

Optionally, the quencher is a dark quencher.

Optionally, the quencher is selected from tetramethyl-rhodamine (TAMRA), dimethylaminoazobenzenesulfonic acid, and a quencher commercially-available under the trademark "Black Hole Quencher" ("BHQ") from Biosearch Technologies, Inc., Novato, CA.

Optionally, the quencher is selected a quencher disclosed in United States Patent No 7019129.

Optionally, the quencher is a quencher of excited state energy having the formula: 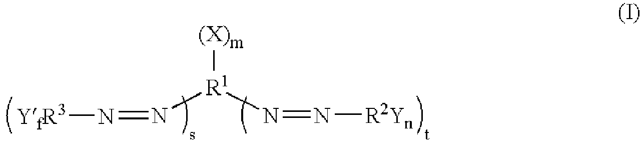 wherein,
R1, R2 and R3 are members independently selected from substituted or unsubstituted aryl, substituted or unsubstituted heteroaryl and substituted or unsubstituted unsaturated alkyl, with the proviso that at least two of R1, R2 and R3 are members selected from substituted or unsubstituted aryl, and substituted or unsubstituted heteroaryl;
X, Y and Y' are members independently selected from reactive functional groups, wherein at least one of X, Y and Y' is: 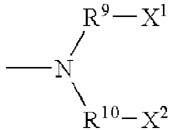 wherein,
   R9 and R10 are members independently selected from alkyl and substituted alkyl; and X1 is a member selected from CH3, -OH, COOH, -NR'R", -SH, and -OP(OX3)(N(X4)2); X2 is OP(OX3)(N(X4)2)
      wherein,
   R', R", are members independently selected from H, and substituted or unsubstituted alkyl; and
   X3 and X4 are members independently selected from substituted and unsubstituted alkyl; f is a number selected from 0 to 4, inclusive, such that when (fxs) is greater than 1, the Y' groups are the same or different;
   m is a number selected from 1 to 4, inclusive, such that when m is greater than 1, the X groups are the same or different;
   n is a number from 0 to 6, inclusive, such that when (nxt) is greater than 1, the Y groups are the same or different;
   s is a number from 1 to 6, inclusive, such that when s is greater than 1 the R3 groups are the same or different; and
   t is a number from 1 to 6, inclusive, such that when t is greater than 1 the R2 groups are the same or different.

Optionally, the quencher is a quencher of excited state energy selected from: 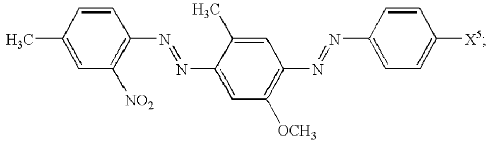 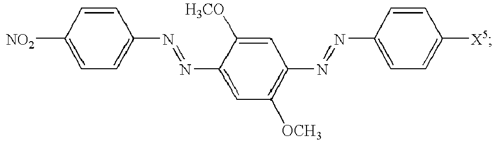 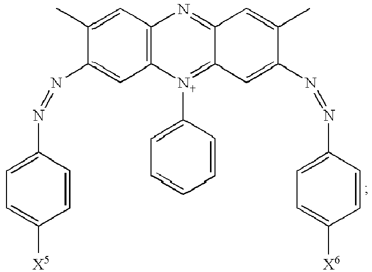 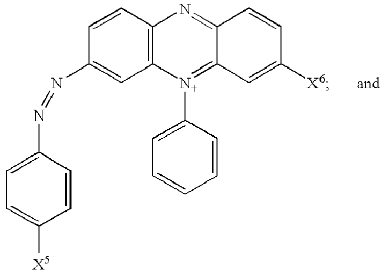 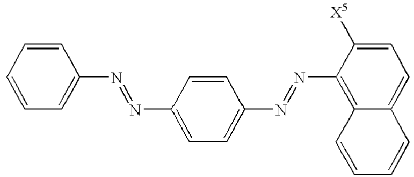 wherein,
X5 and X6 are members independently selected from H, substituted or unsubstituted C1-C6 alkyl, ER', DOOR', -NR'R"-SH, -OP(OX3)N(X4)2, wherein at least one of X5 and X6 is a reactive functional group;
R' is a member selected from the group consisting of H, and alkyl or substituted alkyl;
R" is a member selected from the group consisting of H and substituted alkyl; and
X3 and X4 are members independently selected from CN, and substituted or unsubstituted C1-C6 alkyl.

Optionally, the reporter comprises at least one dye and a cleavage site.

Optionally, the reporter is attached at or adjacent the 5' terminal end of the (e) forward loop primer, and comprises at least one dye and a cleavage site.

Optionally, the reporter is attached at or adjacent the 5' terminal end of the (e) forward loop primer, and comprises first and second dyes located at or adjacent opposing ends of the cleavage site.

Optionally, the reporter is attached at or adjacent the 5' terminal end of the (e) forward loop primer, and comprises at least one dye and at least one quencher located at or adjacent opposing ends of the cleavage site.

Optionally, the reporter is attached at or adjacent the 5' terminal end of the (e) forward loop primer, and comprises at least one dye and at least one quencher located at or adjacent opposing ends of the cleavage site, and the cleavage site is an apurinic/apyrimidinic site (abasic site).

Optionally, the reporter is attached at or adjacent the 5' terminal end of the (e) forward loop primer, and comprises at least one dye selected from 3',6'-dihydroxy-1-oxospiro[2-benzofuran-3,9'-xanthene]-5-carboxylic acid (6-Carboxyfluorescein; 6-FAM); 6-Carboxy-2',7'-dichlorofluorescein diacetate N-succinimidyl ester (6-hexachlorofluorescein; 6-HEX); and 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5) and at least one quencher located at or adjacent opposing ends of the cleavage site, and the cleavage site is an apurinic/apyrimidinic site (abasic site).

Optionally, the reporter is attached at or adjacent the 5' terminal end of the (e) forward loop primer, and comprises at least one dye selected from 3',6'-dihydroxy-1-oxospiro[2-benzofuran-3,9'-xanthene]-5-carboxylic acid (6-Carboxyfluorescein; 6-FAM); 6-Carboxy-2',7'-dichlorofluorescein diacetate N-succinimidyl ester (6-hexachlorofluorescein; 6-HEX); and 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5) and at least one quencher selected from tetramethyl-rhodamine (TAMRA), dimethylaminoazobenzenesulfonic acid, and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the reporter is attached at or adjacent the 5' terminal end of the (e) forward loop primer, and comprises 3',6'-dihydroxy-1-oxospiro[2-benzofuran-3,9'-xanthene]-5-carboxylic acid (6-Carboxyfluorescein; 6-FAM) and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the reporter is attached at or adjacent the 5' terminal end of the (e) forward loop primer, and comprises 6-Carboxy-2',7'-dichlorofluorescein diacetate N-succinimidyl ester (6-hexachlorofluorescein; 6-HEX) and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the reporter is attached at or adjacent the 5' terminal end of the (e) forward loop primer, and comprises 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5) and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the reporter is attached at or adjacent the 5' terminal end of the (f) reverse loop primer, and comprises at least one dye and a cleavage site.

Optionally, the reporter is attached at or adjacent the 5' terminal end of the (f) reverse loop primer, and comprises first and second dyes located at or adjacent opposing ends of the cleavage site.

Optionally, the reporter is attached at or adjacent the 5' terminal end of the (f) reverse loop primer, and comprises at least one dye and at least one quencher located at or adjacent opposing ends of the cleavage site.

Optionally, the reporter is attached at or adjacent the 5' terminal end of the (f) reverse loop primer, and comprises at least one dye and at least one quencher located at or adjacent opposing ends of the cleavage site, and the cleavage site is an apurinic/apyrimidinic site (abasic site).

Optionally, the reporter is attached at or adjacent the 5' terminal end of the (f) reverse loop primer, and comprises at least one dye selected from 3',6'-dihydroxy-1-oxospiro[2-benzofuran-3,9'-xanthene]-5-carboxylic acid (6-Carboxyfluorescein; 6-FAM); 6-Carboxy-2',7'-dichlorofluorescein diacetate N-succinimidyl ester (6-hexachlorofluorescein; 6-HEX); and 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5) and at least one quencher located at or adjacent opposing ends of the cleavage site, and the cleavage site is an apurinic/apyrimidinic site (abasic site).

Optionally, the reporter is attached at or adjacent the 5' terminal end of the (f) reverse loop primer, and comprises at least one dye selected from 3',6'-dihydroxy-1-oxospiro[2-benzofuran-3,9'-xanthene]-5-carboxylic acid (6-Carboxyfluorescein; 6-FAM); 6-Carboxy-2',7'-dichlorofluorescein diacetate N-succinimidyl ester (6-hexachlorofluorescein; 6-HEX); and 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5) and at least one quencher selected from tetramethyl-rhodamine (TAMRA), dimethylaminoazobenzenesulfonic acid, and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the reporter is attached at or adjacent the 5' terminal end of the (f) reverse loop primer, and comprises 3',6'-dihydroxy-1-oxospiro[2-benzofuran-3,9'-xanthene]-5-carboxylic acid (6-Carboxyfluorescein; 6-FAM) and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the reporter is attached at or adjacent the 5' terminal end of the (f) reverse loop primer, and comprises 6-Carboxy-2',7'-dichlorofluorescein diacetate N-succinimidyl ester (6-hexachlorofluorescein; 6-HEX) and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the reporter is attached at or adjacent the 5' terminal end of the (f) reverse loop primer, and comprises 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5) and a quencher commercially-available under the trademark "Black Hole Quencher" ("BHQ") from Biosearch Technologies, Inc., Novato, CA. located at or adjacent opposing ends of the cleavage site, and the cleavage site is an apurinic/apyrimidinic site (abasic site).

Optionally, the isothermal amplification is performed in the presence of an enzyme having polymerase activity.

Optionally, the isothermal amplification is performed in the presence of a polymerase enzyme.

Optionally, the isothermal amplification is performed in the presence of a nucleic acid polymerase enzyme.

Optionally, the isothermal amplification is performed in the presence of a deoxyribonucleic acid polymerase enzyme.

Optionally, the isothermal amplification is performed in the presence of a bacterial deoxyribonucleic acid polymerase enzyme.

Optionally, the isothermal amplification is performed in the presence of a Bacillus deoxyribonucleic acid polymerase enzyme.

Optionally, the isothermal amplification is performed in the presence of a Bacillus stearothermophilus deoxyribonucleic acid polymerase enzyme.

Optionally, the isothermal amplification is performed in the presence of a fragment/subunit of Bacillus stearothermophilus deoxyribonucleic acid polymerase enzyme.

Optionally, the isothermal amplification is performed in the presence of a large fragment/subunit of Bacillus stearothermophilus deoxyribonucleic acid polymerase enzyme.

Optionally, the isothermal amplification is performed in the presence of a large fragment/subunit of Bacillus stearothermophilus deoxyribonucleic acid polymerase enzyme having a reversibly-bound aptamer.

Optionally, the isothermal amplification is performed in the presence of a further enzyme having endonuclease activity.

Optionally, the isothermal amplification is performed in the presence of a further enzyme having apurinic/apyrimidinic endonuclease activity.

Optionally, the isothermal amplification is performed in the presence of a further enzyme having 3'-diesterease activity.

Optionally, the isothermal amplification is performed in the presence of an Endonuclease IV enzyme.

Optionally, the isothermal amplification is performed in the presence of a bacterial Endonuclease IV enzyme.

Optionally, the isothermal amplification is performed in the presence of a Thermus Endonuclease IV enzyme.

Optionally, the isothermal amplification is performed in the presence of a Thermus thermophilus Endonuclease IV enzyme.

Optionally, the isothermal amplification is performed in the presence of a Thermus thermophilus Tth Endonuclease IV enzyme.

Optionally, the isothermal amplification is performed in the presence of a further enzyme having exonuclease activity.

Optionally, the isothermal amplification is performed in the presence of a further enzyme having 3' → 5' exonuclease activity.

Optionally, the isothermal amplification is performed in the presence of a further enzyme having RNase H, 3'-phosphatase and/or AP-endonuclease activity.

Optionally, the isothermal amplification is performed in the presence of an Exonuclease III enzyme.

Optionally, the isothermal amplification is performed in the presence of a bacterial Exonuclease III enzyme.

Optionally, the isothermal amplification is performed in the presence of an Escherichia Exonuclease III enzyme.

Optionally, the isothermal amplification is performed in the presence of an E. coli Exonuclease III enzyme.

Optionally, the isothermal amplification is performed at a temperature of 40°C to 85°C.

Optionally, the isothermal amplification is performed at a temperature of 45°C to 85°C.

Optionally, the isothermal amplification is performed at a temperature of 55°C to 85°C.

Optionally, the isothermal amplification is performed at a temperature of 60°C to 80°C.

Optionally, the isothermal amplification is performed at a temperature of 67°C.

Optionally, the isothermal amplification is performed at a temperature of 65°C.

Optionally, the isothermal amplification is performed at a temperature of 70°C to 80°C.

Optionally, the isothermal amplification is performed at a temperature of 75°C.

Optionally, the isothermal amplification is performed in the presence of a deoxyribonucleic acid polymerase enzyme at a temperature of 67°C. Optionally, the isothermal amplification is performed in the presence of a bacterial deoxyribonucleic acid polymerase enzyme at a temperature of 67°C. Optionally, the isothermal amplification is performed in the presence of a Bacillus deoxyribonucleic acid polymerase enzyme at a temperature of 67°C. Optionally, the isothermal amplification is performed in the presence of a Bacillus stearothermophilus deoxyribonucleic acid polymerase enzyme at a temperature of 67°C. Optionally, the isothermal amplification is performed in the presence of a fragment/subunit of Bacillus stearothermophilus deoxyribonucleic acid polymerase enzyme at a temperature of 67°C. Optionally, the isothermal amplification is performed in the presence of a large fragment/subunit of Bacillus stearothermophilus deoxyribonucleic acid polymerase enzyme at a temperature of 67°C. Optionally, the isothermal amplification is performed in the presence of a large fragment/subunit of Bacillus stearothermophilus deoxyribonucleic acid polymerase enzyme having a reversibly-bound aptamer at a temperature of 67°C.

Optionally, the isothermal amplification is performed in the presence of an Endonuclease IV enzyme at a temperature of 67°C. Optionally, the isothermal amplification is performed in the presence of a bacterial Endonuclease IV enzyme at a temperature of 67°C. Optionally, the isothermal amplification is performed in the presence of a Thermus Endonuclease IV enzyme at a temperature of 67°C. Optionally, the isothermal amplification is performed in the presence of a Thermus thermophilus Endonuclease IV enzyme at a temperature of 67°C. Optionally, the isothermal amplification is performed in the presence of a Thermus thermophilus Tth Endonuclease IV enzyme at a temperature of 67°C.

Optionally, the isothermal amplification is performed for 10-80 minutes.

Optionally, the isothermal amplification is performed for 20-80 minutes.

Optionally, the isothermal amplification is performed for 15-60 minutes.

Optionally, the isothermal amplification is performed for 20-60 minutes.

Optionally, the isothermal amplification is performed for 25-60 minutes.

Optionally, the isothermal amplification is performed for 30-60 minutes.

Optionally, the isothermal amplification is performed for 60 minutes.

Optionally, the isothermal amplification is performed for 20-80 minutes at a temperature of 67°C. Optionally, the isothermal amplification is performed for 30-60 minutes at a temperature of 67°C. Optionally, the isothermal amplification is performed for 60 minutes at a temperature of 67°C.

Optionally, the nucleic acid modification or substitution of the target nucleic acid is a nucleic acid substitution.

Optionally, the nucleic acid modification or substitution of the target nucleic acid is a single-nucleotide polymorphism.

Optionally, the disease is selected from cancer, cardiovascular disorders, diabetes, autoimmune diseases, gastrointestinal disorders, and infectious diseases.

Optionally, the infectious disease is selected from a bacterial infection, a viral infection, and a fungal infection.

Optionally, the infectious disease is a bacterial infection.

Optionally, the infectious disease is a Neisseria, Streptococcus, or Haemophilus infection.

Optionally, the infectious disease is a Neisseria meningitidis, Streptococcus pneumoniae, or Haemophilus influenzae infection.

Optionally, the infectious disease is a Neisseria meningitidis infection, and the set of primers comprises at least one of:
(a) a forward outer primer having a nucleic acid sequence as defined in SEQ ID NO:3;
(b) a reverse outer primer having a nucleic acid sequence as defined in SEQ ID NO:4;
(c) a forward inner primer having a nucleic acid sequence as defined in SEQ ID NO:1;
(d) a reverse inner primer having a nucleic acid sequence as defined in SEQ ID NO:2;
(e) a forward loop primer having a nucleic acid sequence as defined in SEQ ID NO:5 or in SEQ ID NO:6; and
(f) a reverse loop primer having a nucleic acid sequence as defined in SEQ ID NO:7.

Optionally, the infectious disease is a Streptococcus pneumoniae infection, and the set of primers comprises at least one of:
(a) a forward outer primer having a nucleic acid sequence as defined in SEQ ID NO:10;
(b) a reverse outer primer having a nucleic acid sequence as defined in SEQ ID NO:11;
(c) a forward inner primer having a nucleic acid sequence as defined in SEQ ID NO:8;
(d) a reverse inner primer having a nucleic acid sequence as defined in SEQ ID NO:9;
(e) a forward loop primer having a nucleic acid sequence as defined in SEQ ID NO:12; and
(f) a reverse loop primer having a nucleic acid sequence as defined in SEQ ID NO:13.

Optionally, the infectious disease is a Haemophilus influenzae infection, and the set of primers comprises at least one of:
(a) a forward outer primer having a nucleic acid sequence as defined in SEQ ID NO:16;
(b) a reverse outer primer having a nucleic acid sequence as defined in SEQ ID NO:17;
(c) a forward inner primer having a nucleic acid sequence as defined in SEQ ID NO:14;
(d) a reverse inner primer having a nucleic acid sequence as defined in SEQ ID NO:15;
(e) a forward loop primer having a nucleic acid sequence as defined in SEQ ID NO:18; and
(f) a reverse loop primer having a nucleic acid sequence as defined in SEQ ID NO:19.

Optionally, the infectious disease is a fungal infection.

Optionally, the infectious disease is a Candida infection.

Optionally, the infectious disease is a Candida albicans infection.

Optionally, the infectious disease is a Candida albicans infection, and the set of primers comprises at least one of:
(a) a forward outer primer having a nucleic acid sequence as defined in SEQ ID NO:54;
(b) a reverse outer primer having a nucleic acid sequence as defined in SEQ ID NO:55;
(c) a forward inner primer having a nucleic acid sequence as defined in SEQ ID NO:56;
(d) a reverse inner primer having a nucleic acid sequence as defined in SEQ ID NO:57;
(e) a forward loop primer having a nucleic acid sequence as defined in SEQ ID NO:5 or in SEQ ID NO:58; and
(f) a reverse loop primer having a nucleic acid sequence as defined in SEQ ID NO:59.

Optionally, the sample is selected from a cell sample, a tissue sample such as a biopsy sample, and a liquid sample such as a bodily fluid sample.

Optionally, the sample is a liquid sample such as a blood sample or a cerebrospinal fluid (CSF) sample.

Optionally, the step of (a) providing a sample further comprises isolating nucleic acids in the sample.

Optionally, the step of (a) providing a sample further comprises isolating ribonucleic acids in the sample.

Optionally, the step of (a) providing a sample further comprises isolating deoxyribonucleic acids in the sample.

Optionally, the step of (c) performing isothermal amplification comprises contacting the set of primers according to the first aspect of the present invention with the sample.

Optionally, the step of (c) performing isothermal amplification comprises contacting the set of primers according to the first aspect of the present invention with the nucleic acids in the sample.

Optionally, the step of (c) performing isothermal amplification comprises contacting the set of primers according to the first aspect of the present invention with the ribonucleic acids in the sample.

Optionally, the step of (c) performing isothermal amplification comprises contacting the set of primers according to the first aspect of the present invention with the deoxyribonucleic acids in the sample.

Optionally, the step (d) detecting a nucleic acid target in the sample comprises detecting the signal emitted from the reporter.

Optionally, the step (d) detecting a nucleic acid target in the sample comprises detecting the detectable signal emitted from the reporter.

Optionally, the step (d) detecting a nucleic acid target in the sample comprises detecting the fluorescent signal emitted from the reporter.

Optionally, the step (d) detecting a nucleic acid target in the sample comprises detecting the fluorescent signal emitted from the reporter at a wavelength of 495-662 nm.

Optionally, the step (d) detecting a nucleic acid target in the sample comprises detecting the fluorescent signal emitted from the reporter at a wavelength of 495-520 nm.

Optionally, the step (d) detecting a nucleic acid target in the sample comprises detecting the fluorescent signal emitted from the reporter at a wavelength of 535-565 nm.

Optionally, the step (d) detecting a nucleic acid target in the sample comprises detecting the fluorescent signal emitted from the reporter at a wavelength of 646-662 nm.

Optionally, the reporter comprises 3',6'-dihydroxy-1-oxospiro[2-benzofuran-3,9'-xanthene]-5-carboxylic acid (6-Carboxyfluorescein; 6-FAM) and the step (d) detecting a nucleic acid target in the sample comprises detecting the fluorescent signal emitted from the reporter at a wavelength of 495-520 nm.

Optionally, the reporter comprises 6-Carboxy-2',7'-dichlorofluorescein diacetate N-succinimidyl ester (6-hexachlorofluorescein; 6-HEX) and the step (d) detecting a nucleic acid target in the sample comprises detecting the fluorescent signal emitted from the reporter at a wavelength of 535-565 nm.

Optionally, the reporter comprises 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5) and the step (d) detecting a nucleic acid target in the sample comprises detecting the fluorescent signal emitted from the reporter at a wavelength of 646-662 nm.

Optionally, a presence of a signal emitted from the reporter indicates a presence of the nucleic acid target in the sample.

Optionally, an absence of a signal emitted from the reporter indicates an absence of the nucleic acid target in the sample.

Optionally, a presence of a signal emitted from the reporter indicates an absence of the nucleic acid modification or substitution of the target nucleic acid in the sample.

Optionally, an absence of a signal emitted from the reporter indicates a presence of the nucleic acid modification or substitution of the target nucleic acid in the sample.

Optionally, the presence of the nucleic acid target in the sample indicates the presence of a disease or disorder.

Optionally, the presence of the nucleic acid modification or substitution of the target nucleic acid in the sample indicates the presence of a disease or disorder associated with the nucleic acid modification or substitution of the target nucleic acid.

Optionally, the method for identifying a target nucleic acid comprises identifying the nucleic acid target in the sample when the nucleic acid target is present in the sample.

Optionally, the method for identifying a target nucleic acid comprises identifying the nucleic acid target in the sample when the sample comprises less than 10⁵ copies of the nucleic acid target.

Optionally, the method for identifying a target nucleic acid comprises identifying the nucleic acid target in the sample when the sample comprises less than 10³ copies of the nucleic acid target.

Optionally, the method for identifying a target nucleic acid comprises identifying the nucleic acid target in the sample when the sample comprises less than 10² copies of the nucleic acid target.

Optionally, the method for identifying a nucleic acid modification or substitution of a target nucleic acid comprises identifying the nucleic acid modification or substitution of the target nucleic acid in the sample when the nucleic acid target is present in the sample.

Optionally, the method for identifying a nucleic acid modification or substitution of a target nucleic acid comprises identifying the nucleic acid modification or substitution of the target nucleic acid in the sample when the sample comprises less than 10⁵ copies of the nucleic acid target.

Optionally, the method for identifying a nucleic acid modification or substitution of a target nucleic acid comprises identifying the nucleic acid modification or substitution of the target nucleic acid in the sample when the sample comprises less than 10³ copies of the nucleic acid target.

Optionally, the method for identifying a nucleic acid modification or substitution of a target nucleic acid comprises identifying the nucleic acid modification or substitution of the target nucleic acid in the sample when the sample comprises less than 10² copies of the nucleic acid target.

### Brief Description of the Drawings

Embodiments of the invention will now be described with reference to the accompanying drawings in which:

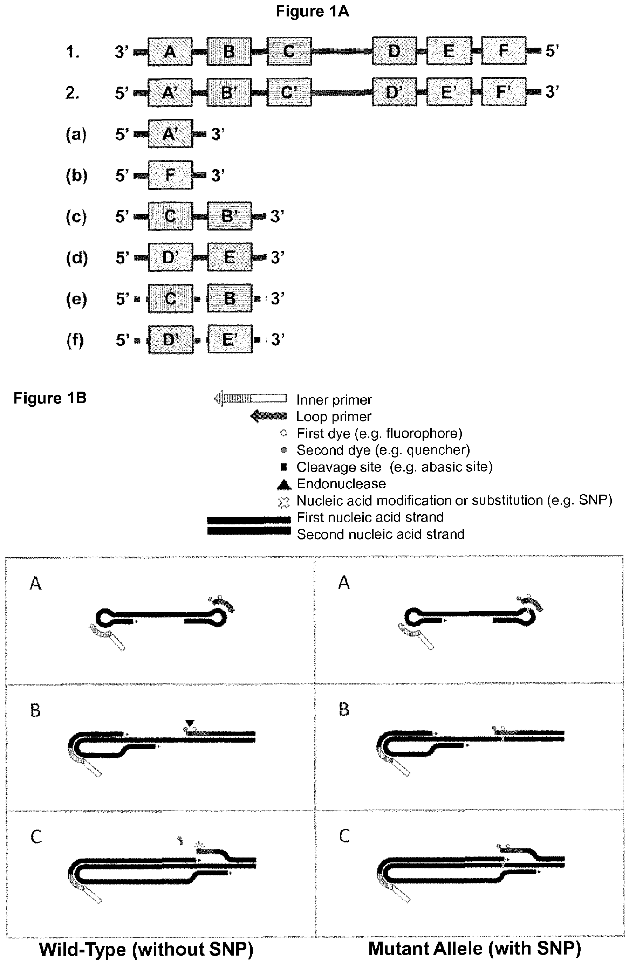

**Figure 1A** is a schematic diagram of a set of primers of the invention comprising (a) a forward outer primer; (b) a reverse outer primer; (c) a forward inner primer; (d) a reverse inner primer, (e) a forward loop primer and (f) a reverse loop primer; wherein each primer is capable of binding to a target nucleic acid having complementary first (1.) and second (2.) nucleic acid strands, wherein the first (1.) nucleic acid strand has first (A), second (B), third (C), fourth (D), fifth (E) and sixth (F) regions; and wherein the second (2.) nucleic acid strand has first (F'), second (E'), third (D'), fourth (C'), fifth (B') and sixth (A') regions; wherein the fourth (D), fifth (E) and sixth (F) regions of the first (1.) nucleic acid strand are complimentary to the third (D'), second (E'), and first (F') regions of the second (2.) nucleic acid strand; and wherein the fourth (C'), fifth (B') and sixth (A') regions of the second (2.) nucleic acid strand are complimentary to the third (C), second (B), and first (A) regions of the first (1.) nucleic acid strand; and wherein (a) the forward outer primer is complementary to the first region (A) of the first (1.) nucleic acid strand; (b) the reverse outer primer is complementary to the first (F') region of the second (2.) nucleic acid strand; (c) the forward inner primer has first and second parts, wherein: (i) the first part is complementary to the second region (B) of the first (1.) nucleic acid strand; and (ii) the second part is complementary to the fourth (C') region of the second (2.) nucleic acid strand; and (d) the reverse inner primer has first and second parts, wherein: (i) the first part is complementary to the second (E') region of the second (2.) nucleic acid strand; and (ii) the second part is complementary to the fourth (D) region of the first (1.) nucleic acid strand; (e) the forward loop primer is complementary to a region between the fourth (C') and fifth (B') regions of the second (2.) nucleic acid strand; (f) the reverse loop primer is complementary to a region between the fourth (D) and fifth (E) regions of the first (1.) nucleic acid strand;
**Figure 1B** is a schematic diagram of an isothermal amplification using the primers of the invention; wherein (A) loop regions of the double-looped LAMP template, produced by strand-displacing polymerase extension from the outer and inner primers, are targeted by the inner primer and primer/probe; (B) after primer and probe target hybridisation, strand-displacement polymerase extension initiates, unwinding both loop structures, and primer/probe target hybridisation produces a dsDNA abasic site initiating Endonuclease IV cleavage in the wild-type reaction, and presence of the SNP in the mutant allele reaction inhibits abasic site dsDNA formation, preventing cleavage; and (C) combination of reaction temperature and strand-displacement polymerase extension causes fluorophore and quencher dissociation in the wild-type reaction, producing fluorescence, wherein the mutant allele reaction, the fluorophore and quencher remain associated, preventing fluorescence production and enabling wild-type and mutant allele differentiation;

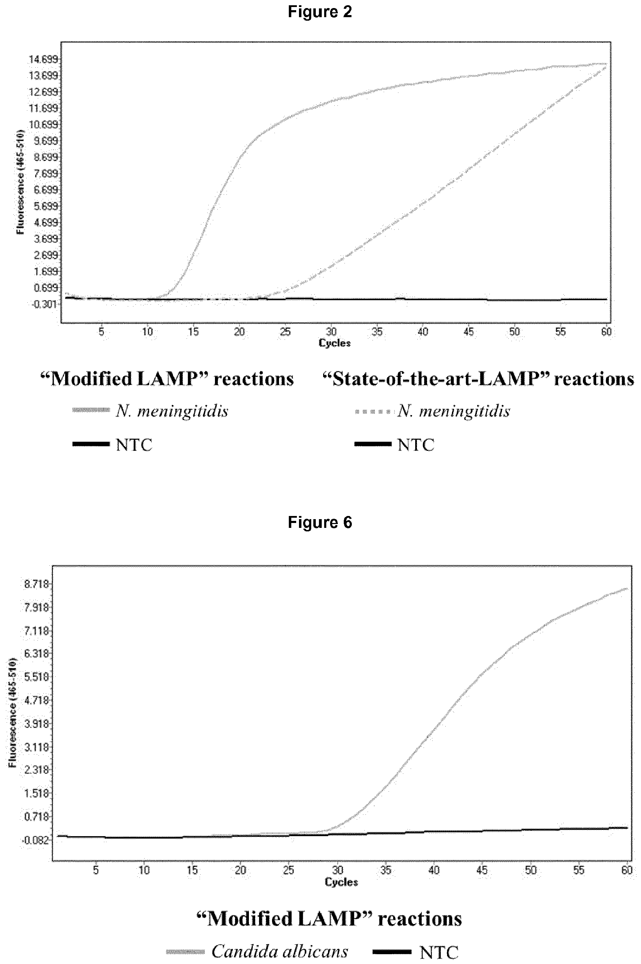

**Figure 2** shows the results of a singleplex *N. meningitidis* assay single-target detection using the primers of the invention (modified LAMP) compared to detection using the primers of the state of the art (state-of-the-art LAMP), wherein the singleplex modified LAMP (grey) and state-of-the-art LAMP (red) *N. meningitidis* assays were challenged with *N. meningitidis* genomic DNA at 10³ copies, no template control (NTC) reactions (black) were performed in parallel, resulting LAMP fluorescence signal was recorded in the LightCycler® 480 FAM detection channel, with representative amplification curves for each reaction shown, successful modified LAMP and state-of-the-art LAMP detection of *N. meningitidis* is observed, however, modified LAMP produced earlier time-to-detection and increased fluorescence levels compared to state-of-the-art LAMP, and the NTC reactions performed successfully as no detection was observed;

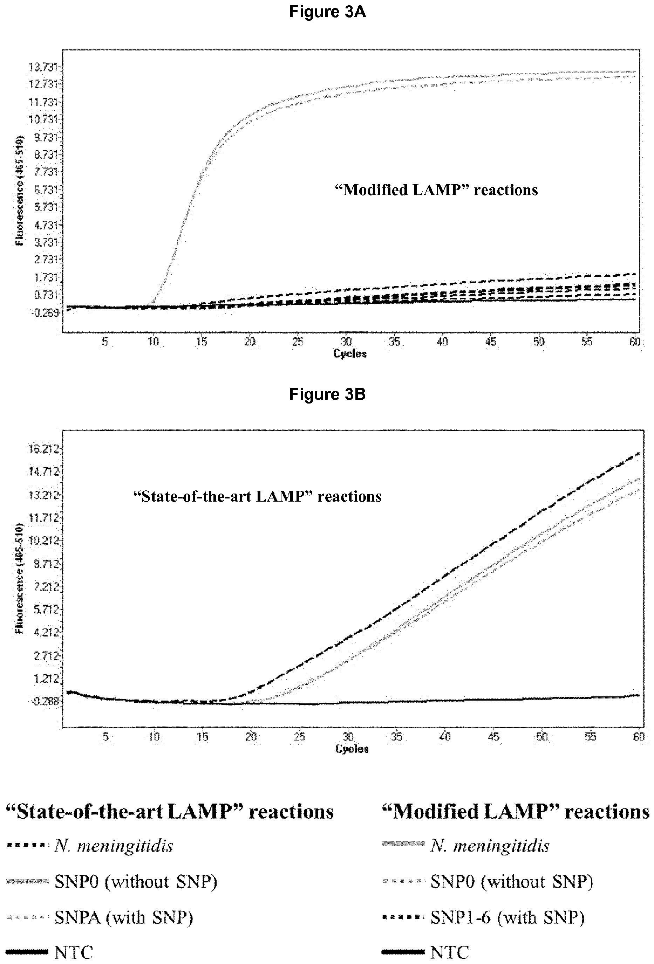

**Figure 3** shows the results of a singleplex modified LAMP *N. meningitidis* assay SNP identification with comparison to state-of-the-art LAMP, wherein the singleplex modified LAMP and state-of-the-art LAMP *N. meningitidis* assays were separately challenged with templates without SNPs, *N*. *meningitidis* genomic DNA (grey) and SNPO (red), at 10⁵ copies, in parallel, the modified LAMP and state-of-the-art LAMP assays were also challenged with templates containing SNPs, SNP1-6 (A, dashed black) and SNPA (B, dashed black) respectively, at 10⁵ copies, no template control (NTC) reactions were performed in parallel (black), resulting amplification curves indicate modified LAMP single-base specificity as only templates without SNPs were detected, single-base specificity in the state-of-the-art LAMP assay was not observed as all templates, with or without SNPs, were detected, and the NTC reactions performed successfully as no detection was observed;

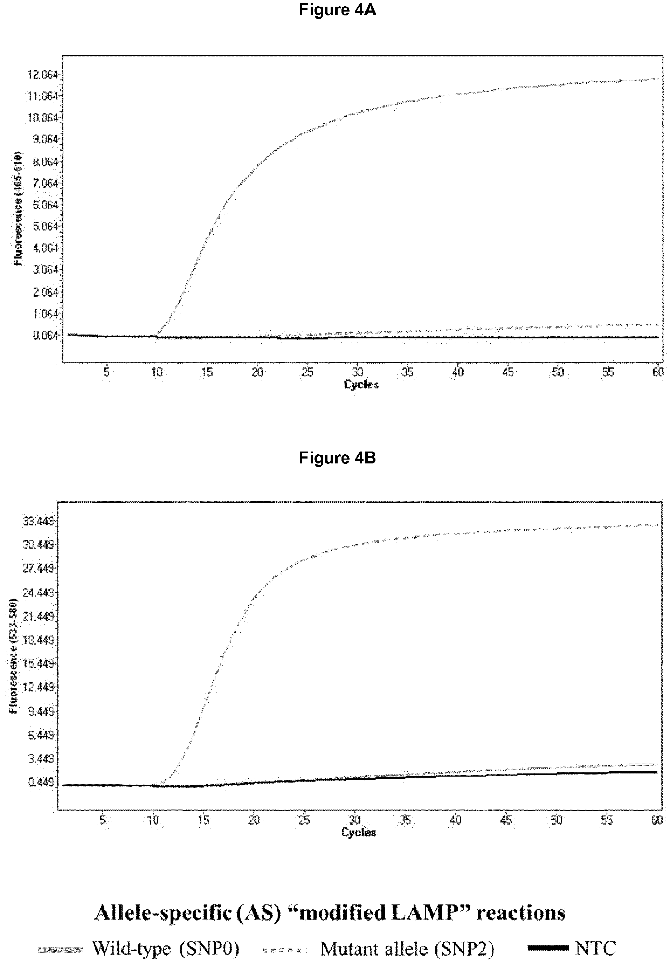

**Figure 4** shows the allele-specific (AS) modified LAMP *N. meningitidis* assay single-tube detection of either wild-type or mutant allele templates, wherein the AS modified LAMP *N. meningitidis* assay was separately challenged with the wild-type template SNPO (grey), and the mutant allele template SNP2 (red), at 10⁵ copies, a NTC reaction was performed in parallel (black), resulting LAMP fluorescence signal was recorded in the LightCycler® 480 FAM (wild-type) and HEX (mutant allele) detection channels, with representative amplification curves for each reaction shown, successful modified LAMP detection of both templates was observed in respective detection channels only, with no unspecific detection of either template in non-corresponding channels observed, and the NTC reaction performed successfully as no detection was observed;

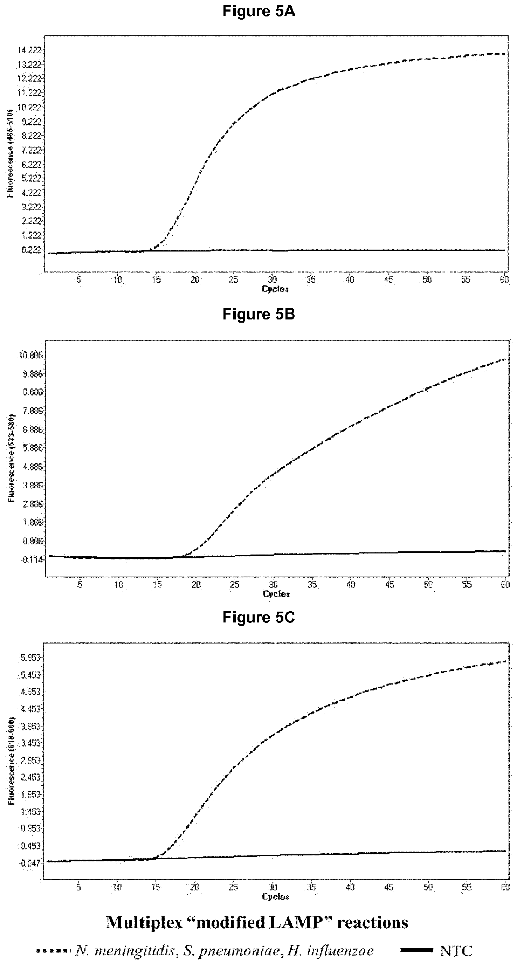

**Figure** 5 shows multiplex modified LAMP *N. meningitidis, S. pneumoniae* and *H. influenzae* assay simultaneous multiple-target detection, wherein the multiplex modified LAMP assay was challenged with three bacterial templates, *N. meningitidis* (A, dashed black), S. *pneumoniae* (B, dashed black) and *H. influenzae* (C, dashed black), at 10² genome copies in a single reaction, a NTC reaction was also performed in parallel (black), resulting LAMP fluorescence signal was recorded in the LightCycler® 480 FAM (*N. meningitidis*), HEX (*S*. *pneumoniae*) and Cy5 (*H. influenzae*) detection channels with representative amplification curves for each reaction shown, and successful simultaneous detection of all three bacterial templates, with no amplification in the NTC control reaction, was observed; and
**Figure 6** shows single-target detection of C. albicans bacterial template at 10^5 genome copies was successfully demonstrated using the singleplex C. albicans modified LAMP assay (Figure 6, grey). The NTC reaction performed successfully as no amplification was observed (Figure 6, black).

### Examples

Embodiments of the invention will now be described with reference to the following non-limiting examples:

### Materials and Methods

### Bacterial and Fungal DNA Template Preparation

The singleplex modified LAMP *N. meningitidis* assay was evaluated using a range of *N. meningitidis, Neisseria* and closely related non-Neisseria reference strains (Table 1). The multiplex modified LAMP *N. meningitidis, S. pneumoniae* and *H. influenzae* assay was performed using type-strains *N*. *meningitidis* NCTC 10025, *S*. *pneumoniae* DSM 20566 and *H. influenzae* DSM 4690. The singleplex modified LAMP *Candida alibicans* assay was performed using *Candida alibicans* CBS 562 type strain. All bacterial and fungal strains, stored at -80°C, were cultured in brain heart infusion (BHI) media (Oxoid, Hampshire, UK) at 37°C for 18 h under microaerophilic conditions, excluding *Haemophilus* strains which were cultured using Haemophilus test media (Oxoid). DNA extractions were performed using the DNeasy Blood and Tissue kit (Qiagen, Hilden, Germany) followed by DNA quantification using the Qubit dsDNA broad range/high sensitivity assay kits and Qubit 2.0 fluorometer (Life Technologies, Warrington, UK). Genome size standards of 2.2 Mb, 2.1 Mb, 1.83 Mb, and 15.5 Mb for *N. meningitidis, S. pneumoniae, H. influenzae,* and *Candida alibicans,* respectively, were used to convert resulting DNA concentrations to genome copy values. Extracted DNA samples were stored at -80°C prior to use.

| **Table 1: Singleplex modified LAMP *Neisseria meningitidis* assay specificity panel.** | | |
|---|---|---|
| Organism | Strain | modified LAMP Result |
| **Inclusivity Panel** | | |
| *N. meningitidis* reference strains | | |
| *N. meningitidis* (A, type strain) | NCTC 10025 | + |
| *N. meningitidis* (A) | DSM 10036 | + |
| *N. meningitidis* (A) | NCTC 3372 | + |
| *N. meningitidis* (A) | NCTC 3375 | + |
| *N. meningitidis* (B) | ATCC 13090 | + |
| *N. meningitidis* (C) | ATCC 13102 | + |
| *N. meningitidis* (C) | DSM 15464 | + |
| *N. meningitidis* (W) | NCTC 11203 | + |
| *N. meningitidis* (X) | NCTC 10790 | + |
| *N. meningitidis* (Y) | NCTC 10791 | + |

| **Exclusivity Panel** | | |
|---|---|---|
| *Neisseria* reference strains and closely related non-Neisseria reference strains | | |
| *N. animalis* | DSM 23392 | - |
| *N. animaloris* | DSM 21642 | - |
| *N. bacilliformis* | DSM 23338 | - |
| *N. canis* | DSM 18000 | - |
| *N. caviae* | DSM 23336 | - |
| *N. cuniculi* | DSM 21768 | - |
| *N. dentiae* | DSM 19151 | - |
| *N. elongata subsp. elongata* | DSM 17712 | - |
| *N. elongata subsp. glycolytica* | DSM 23337 | - |
| *N. elongata subsp. nitroreducens* | DSM 17632 | - |
| *N. flavescens* | DSM 17633 | - |
| *N. gonorrhoeae* | ATCC 19424 | - |
| *N. gonorrhoeae* | DSM 9188 | - |
| *N. gonorrhoeae* | DSM 9189 | - |
| *N. lactamica* | ATCC 23970 | - |
| *N. lactamica* | DSM 4691 | - |
| *N. macacae* | DSM 19175 | - |
| *N. mucosa* | DSM 17611 | - |
| *N. ovis* | DSM 18075 | - |
| *N. perflava* | DSM 18009 | - |
| *N. polysaccharea* | DSM 22809 | - |
| *N. shayeganii* | DSM 22246 | - |
| *N. sicca* | DSM 17713 | - |
| *N. subflava* | DSM 17610 | - |
| *N. wadsworthii* | DSM 22247 | - |
| *N. weaveri* | DSM 17688 | - |
| *N. zoodegmatis* | DSM 21483 | - |
| *N. zoodegmatis* | DSM 21643 | - |
| *H. influenzae* | DSM 4690 | - |
| *H. parainfluenzae* | DSM 8978 | - |
| *H. haemolyticus* | CCUG 15312 | - |
| *H. somnus* | CCUG 12839 | - |
| *S. pneumoniae* | DSM 20566 | - |
| *S. pseudopneumoniae* | DSM 18670 | - |
| *S. agalactiae* | BCCM 15081 | - |
| *S. mitis* | DSM 12643 | - |
| *K. pneumoniae* | DSM 30104 | - |
| *P. aeruginosa* | DSM 50071 | - |
| *E. coli* | DSM 30083 | - |
| *E. faecalis* | DSM 20371 | - |
| *S*. *aureus* | DSM 346 | - |
| NCTC, National Collection of Type Cultures; DSM, Leibniz Institute DSMZ - German Collection of Microorganisms and Cell Cultures; ATCC, American Type Culture Collection; CCUG, Culture Collection, University of Göteborg, Sweden; BCCM, Belgian Coordinated Collections of Microorganisms; +, positive; -, negative. | | |

### Diagnostic targets and modified LAMP Oligonucleotides

N. meningitidis, S. pneumoniae, *H. influenzae,* and C. albicans state-of-the-art LAMP oligonucleotides, designed with PrimerExplorer V4 (Eiken Chemical) using diagnostic targets *NMO_1242, SPNA45_01710* and *pstA,* respectively (Higgins, O.; Clancy, E.; Cormican, M.; Boo, T. W.; Cunney, R.; Smith, T. J., Evaluation of an Internally Controlled Multiplex Tth Endonuclease Cleavage Loop-Mediated Isothermal Amplification (state-of-the-art LAMP) Assay for the Detection of Bacterial Meningitis Pathogens. International journal of molecular sciences 2018, 19, (2), 524) were modified to create the modified LAMP and state-of-the-art LAMP oligonucleotides used in this study (Table 2). Oligonucleotide modifications included design of new reverse loop primers for the *S*. *pneumoniae* and *H. influenzae* assays, with addition of two 5'-end thymine residues to the forward loop primer of the *N. meningitidis* assay. Standard desalted oligonucleotides were synthesised by Integrated DNA Technologies. The modified LAMP primer/probes for *N. meningitidis, S. pneumoniae, H. influenzae,* and C. albicans labelled with FAM, HEX, Cy5 and FAM fluorophores, respectively, and the state-of-the-art LAMP primer/probe labelled with a FAM fluorophore, were HPLC purified and synthesised by Metabion International AG (Planegg, Germany). Each fluorophore corresponded to one of three detection channels of the LightCycler® 480 instrument II (Roche Diagnostics, Sussex, UK) used to perform the modified LAMP reactions.

| **Table 2: modified LAMP and state-of-the-art LAMP oligonucleotides.** | |
|---|---|
| **Primer Type** | **Sequence (5'-3')** |
| ***N. meningitidis* modified LAMP** | |
| Forward Inner | TGTCGGTGGCTTTGTTGGTGGTGTCGC- GTGCAAACAGATACGTCCG |
| Reverse Inner | CCGATGTACCAGCACCTTGTCC- GTTTGCGCTGATTACGCCTC |
| Forward Outer | CCCAATTCCACATCAATACGTG |
| Reverse Outer | GTGGTGTCGGTGGTGTTG |
| modified LAMP Primer/Probe Wild-Type | (BHQ1)TTGA(dSpacer)**A**(FAM-dT)TGTGTTGGGCGGTTTG |
| modified LAMP Primer/Probe Mutant (SNP2 template specific) | (BHQ1)TTGA(dSpacer)**C**(HEX-dT)TGTGTTGGGCGGTTTG |
| Reverse Loop | CACCACTTGGAAAAACAGAGGC |

| ***S. pneumoniae* modified LAMP** | |
|---|---|
| Forward Inner | TGGAAAATGCTCTGGCTTTTGAAGTGA- CCTACACCAATATCCTCGCT |
| Reverse Inner | TCTGTCTGGTAGACAGAATGACGGA- TCTTTGAGAATCAGATGCTGGA |
| Forward Outer | TCCGTCAACGAGGCACAA |
| Reverse Outer | AGCAAACTCACCAAGCGC |
| Forward Loop | TGATGAAACAGACAAGCTGATTCT |
| modified LAMP | (BHQ1)ACTC(dSpacer)CA(HEX- |
| Primer/Probe | dT)GCGCAATGATGGTATAATCC |

| ***H. influenzae* modified LAMP** | |
|---|---|
| Forward Inner | TGCCGCTGCTTCACGTAAATTATTTGG- TGCTTATTCCTATCGTGGTACG |
| Reverse Inner | CTTGGTTGCTCTCAATGGCAAG- GCACGCCAGTTAAAATCCCT |
| Forward Outer | GGCTGGAGCATTCGCATT |
| Reverse Outer | TTCTCCTGAAATTCGGGCAA |
| Forward Loop | AACATATTGTCCGTAGTGCG |
| modified LAMP Primer/Probe | (BHQ2)TTGT(dSpacer)A(Cy5-dT)CGAGCAGCTAAATCAGGGA |

| ***C. albicans* modified LAMP** | |
|---|---|
| Forward Inner | TGATGTGGTTGTGGTTGTGGTTGCGATATCAACTTTTATACA CCCATGT |
| Reverse Inner | ACAGCATCAACCTGAATTACAAAACACTACTGATGTTGTAGC ATTTGCTG |
| Forward Outer | CACCAGAATTTAGGTGCCATG |
| Reverse Outer | GCGGAGTTGATTGTTTTGGTTG |
| modified LAMP Primer/Probe | BHQ 1 -TTCA(d Space r)C(FAM-dT)TGTACTGGAAG CTCGT |
| Reverse Loop | CCAACAAATTAAACACATTCAACAGC |

| ***N. meningitidis* state-of-the-art LAMP** | |
|---|---|
| state-of-the-art LAMP Primer/Probe | (FAM)TGTC(dSpacer)G(BHQ1- dT)GGCTTTGTTGGTGGTGTCGC- GTGCAAACAGATACGTCCG |
| Forward Inner | TGTCGGTGGCTTTGTTGGTGGTGTCGC- GTGCAAACAGATACGTCCG |
| Reverse Inner | CCGATGTACCAGCACCTTGTCC- GTTTGCGCTGATTACGCCTC |
| Forward Outer | CCCAATTCCACATCAATACGTG |
| Reverse Outer | GTGGTGTCGGTGGTGTTG |
| Forward Loop | TTGAGATTGTGTTGGGCGGTTTG |
| Reverse Loop | CACCACTTGGAAAAACAGAGGC |
| -, separation between 5' antisense and 3' sense inner primer sequences; BHQ1-dT, black hole quencher 1 linked to thymine; dSpacer, 1',2'-dideoxyribose; FAM, 6-carboxyfluorescein fluorophore; HEX, 6-hexachlorofluorescein fluorophore; Cy5, cyanine fluorophore. | |

Singleplex modified LAMP N. meningitidis assay single-target detection with comparison to state-of-the-art LAMP

The singleplex modified LAMP *N. meningitidis* assay reaction contained 1×Isothermal Amplification Buffer (New England Biolabs, Hitchin, UK), 6 mM MgSO₄ (Roche Diagnostics), 1.4 mM deoxynucleotide triphosphate set (New England Biolabs), *N. meningitidis* oligonucleotides [1.6 µM forward and reverse inner, 0.4 µM modified LAMP primer/probe wild-type and reverse loop, 0.2 µM forward and reverse outer], 8 U *Bst* 2.0 WarmStart DNA polymerase (New England Biolabs), 1 U Endonuclease IV (New England Biolabs), 1 µL DNA template or 1 µL molecular grade water for NTC reaction, and molecular grade water to give a final reaction volume of 25 µL. The singleplex state-of-the-art LAMP *N. meningitidis* assay was prepared as per the modified LAMP assay, with modifications: the Endonuclease IV enzyme was replaced with 15 U *Tth* Endonuclease IV (New England Biolabs); the modified LAMP primer/probe wild-type was replaced with unmodified forward loop primer; and 0.8 µM of the forward inner primer was replaced with state-of-the-art LAMP primer/probe (Table 2). Reactions were performed for 60 x 1 min cycles at 67°C in a LightCycler® 480 instrument II (Roche Diagnostics). The fluorescence detection channel used was 495-520 nm (FAM) with fluorescent measurements recorded every min. Single-target detection using the singleplex modified LAMP and state-of-the-art LAMP *N. meningitidis* assays was demonstrated by challenging both assays with 10³ copies of type-strain *N. meningitidis* genomic DNA (Figure 2). No template control (NTC) reactions using molecular grade water in place of bacterial template were carried out in parallel. Positive results in each reaction were recorded on the LightCycler® 480 as exponential signal acquisition exceeding background fluorescence and represented as fluorescence amplification curves. Cycle threshold (Ct) values denoted cycles at which fluorescent signal exceeded background levels. As reactions were performed for 60 x 1 min cycles, resulting Ct-values acted as approximate time-to-positivity values in minutes.

Singleplex modified LAMP N. meningitidis assay analytical specificity, sensitivity and clinical sample testing

All clinical samples were collected and processed by the IMSRL during routine diagnostic services in accordance with ethical review committee approved protocols. Samples were not analysed for human DNA in this study, and thus, the Ethics Committee of the National University of Ireland, Galway deemed that ethical approval for the evaluation of these samples was not required.

Analytical specificity of the singleplex modified LAMP *N. meningitidis* assay was evaluated using genomic DNA from a panel of bacterial reference strains (Table 1) at 10⁵ genome copy concentrations. The limit of detection (LOD) of the singleplex modified LAMP *N. meningitidis* assay was determined by testing 6 replicates of 32, 16, 8, 4, 2 and 1 genome copy concentrations of type-strain *N. meningitidis* NCTC 10025 genomic DNA. Probit regression analysis was performed on the resulting data using Minitab 17 (Table 3) to establish assay LOD with 95% probability. The clinical application of the singleplex modified LAMP *N. meningitidis* assay was assessed using archived genomic DNA extracted from blood and cerebrospinal fluid (CSF) samples of confirmed bacterial meningitis cases. The Irish Meningitis and Sepsis Reference Laboratory (IMSRL) supplied 72 anonymised samples which were previously collected and processed as part of routine diagnostic service. IMSRL DNA extractions were carried out using a QIAsymphony SP/AS instrument with QIAamp DSP DNA Blood Mini Kits (Qiagen), as per manufacturer instructions, followed by real-time PCR analysis for *N. meningitidis, S. pneumoniae* and *H. influenzae.* We reconfirmed the presence of *N. meningitidis, S. pneumoniae* and *H. influenzae* in each respective sample using singleplex real-time PCR assays targeting the *N. meningitidis NMO_1242, S. pneumoniae lepA* and *H. influenzae pstA* genes (Table 4). PCR reactions were performed on a LightCycler® 480 II instrument, using the LightCycler® 480 Probes Master kit (Roche Diagnostics) as per manufactures instructions, testing 2.5 µL of each sample (Table 5). For comparative purposes, the diagnostic sensitivity and specificity of the singleplex modified LAMP *N. meningitidis* assay was also determined by testing 2.5 µL of each sample (Table 5). Samples from cases of meningococcal infection were used to determine modified LAMP diagnostic sensitivity, and samples from cases of pneumococcal and Haemophilus infection were used to determine modified LAMP diagnostic specificity. Positive control reactions incorporating respective type-strain genomic DNA at 10³ genome copies, and negative control reactions substituting molecular grade water for bacterial template, were carried out in parallel to the above reactions.

| **Table 3: Singleplex modified LAMP *N. meningitidis* assay limit-of-detection (LOD) Probit analysis.** | |
|---|---|
| Genome copy concentration tested | Replicates tested / Replicates detected |
| 32 | 6 / 6 |
| 16 | 6 / 6 |
| 8 | 6 / 6 |
| 4 | 6 / 6 |
| 2 | 6 / 4 |
| 1 | 6 / 1 |
| **Genome copy LOD per reaction (95% probability)** | **3.1** |

| **Table 4: PCR oligonucleotides.** | |
|---|---|
| **Type** | **Sequence (5'-3')** |
| ***N. meningitidis*** | |
| Forward | CGACATGTTCGAACGTAATCTCC |
| Probe | (FAM)TATCGGGCAAAGCCAAATGCGAAG(BHQ1) |
| Reverse | ATTTCGGTGGCGCGTTT |

| ***S. pneumoniae*** | |
|---|---|
| Forward | CTCGT AAGCGT AAACTCCTTG |
| Probe | (FAM)ACGCATGAAATCCATCGGATCAGTT(BHQ1) |
| Reverse | CATACTCAAGACGCTGAGGA |

| ***H. influenzae*** | |
|---|---|
| Forward | GGTACGCACYACGGACAATATG |
| Probe | (FAM)AGCTCTTGGTTGCTCTCAATGGCA(BHQ1) |
| Reverse | CCTGATTTAGCYGCTCGATAACA |
| FAM, 6-carboxyfluorescein fluorophore; BHQ1, black hole quencher | |

**Table 5: Singleplex modified LAMP N. meningitidis assay clinical sample testing using N. meningitidis, S. pneumoniae and H. influenzae PCR-positive DNA extracts from blood and CSF specimens of confirmed bacterial meningitis cases.**

| ***N. meningitidis*** | | | |
|---|---|---|---|
| **Clinical Samples** | | | |
| Sample No. | Clinical Specimen | *N. meningitidis* PCR | ***N. meningitidis* modified LAMP** |
| | | (Ct Value) | **(Ct Value)** |
| 1 | BLD | 36.97 | 24.30 |
| 2 | BLD | 30.82 | 15.06 |
| 3 | BLD | 31.46 | 14.49 |
| 4 | BLD | 30.02 | 15.47 |
| 5 | BLD | 30.05 | 15.05 |
| 6 | BLD | 34.17 | 16.26 |
| 7 | CSF | 33.55 | 17.23 |
| 8 | BLD | 36.11 | 37.13 |
| 9 | CSF | 30.21 | 13.28 |
| 10 | BLD | 33.06 | 16.82 |
| 11 | CSF | 30.32 | 15.27 |
| 12 | BLD | 33.17 | 17.62 |
| 13 | BLD | 36.37 | 21.37 |
| 14 | BLD | 33.47 | 17.73 |
| 15 | BLD | 23.82 | 12.01 |
| 16 | BLD | 36.72 | 17.86 |
| 17 | BLD | 33.91 | 16.00 |
| 18 | CSF | 36.46 | 23.05 |
| 19 | BLD | 29.88 | 14.62 |
| 20 | BLD | 34.83 | 18.28 |
| 21 | BLD | 33.89 | 17.37 |
| 22 | BLD | 36.06 | 12.23 |
| 23 | CSF | 34.02 | 16.84 |
| 24 | CSF | 23.98 | 13.29 |
| 25 | CSF | 32.79 | 18.20 |
| 26 | CSF | 23.04 | 13.53 |
| 27 | CSF | 33.31 | 18.49 |
| 28 | CSF | 22.39 | 13.27 |
| 29 | CSF | 27.29 | 13.65 |
| 30 | CSF | 26.53 | 12.60 |
| 31 | CSF | 22.98 | 13.75 |
| 32 | CSF | 30.30 | 14.52 |
| 33 | CSF | 26.93 | 12.11 |

| ***S. pneumoniae*** | | | |
|---|---|---|---|
| **Clinical Samples** | | | |
| Sample No. | Clinical Specimen | *S. pneumoniae* PCR | ***N. meningitidis* modified LAMP** |
| | | (Ct Value) | **(Ct Value)** |
| 34 | BLD | 31.61 | - |
| 35 | CSF | 24.13 | - |
| 36 | BLD | 28.01 | - |
| 37 | CSF | 34.64 | - |
| 38 | CSF | 26.33 | - |
| 39 | BLD | 28.59 | - |
| 40 | CSF | 30.47 | - |
| 41 | CSF | 33.22 | - |
| 42 | CSF | 31.99 | - |
| 43 | CSF | 25.32 | - |
| 44 | BLD | 31.84 | - |
| 45 | BLD | 26.87 | - |
| 46 | CSF | 29.31 | - |
| 47 | BLD | 27.05 | - |
| 48 | BLD | 32.61 | - |
| 49 | CSF | 25.60 | - |
| 50 | CSF | 23.18 | - |
| 51 | CSF | 28.83 | - |
| 52 | CSF | 23.61 | - |
| 53 | CSF | 26.34 | - |
| 54 | CSF | 31.06 | - |
| 55 | CSF | 28.81 | - |

| ***H. influenzae*** | | | |
|---|---|---|---|
| **Clinical Samples** | | | |
| Sample No. | Clinical Specimen | *H. influenzae* PCR (Ct Value) | ***N. meningitidis* modified LAMP** |
| | | | **(Ct Value)** |
| 56 | BLD | 38.50 | - |
| 57 | CSF | 37.26 | - |
| 58 | BLD | 37.48 | - |
| 59 | CSF | 36.54 | - |
| 60 | CSF | 32.24 | - |
| 61 | CSF | 28.65 | - |
| 62 | CSF | 27.95 | - |
| 63 | BLD | 35.50 | - |
| 64 | BLD | 38.40 | - |
| 65 | BLD | 37.73 | - |
| 66 | BLD | 34.44 | - |
| 67 | BLD | 38.61 | - |
| 68 | BLD | 31.18 | - |
| 69 | BLD | 38.45 | - |
| 70 | BLD | 37.13 | - |
| 71 | CSF | 35.73 | - |
| 72 | CSF | 30.99 | - |

| | | | |
|---|---|---|---|
| BLD, blood; CSF, cerebrospinal fluid; -, negative. | | | |

Singleplex modified LAMP N. meningitidis assay SNP identification with comparison to state-of-the-art LAMP

Templates used to demonstrate modified LAMP single-base specificity were synthetic 500 bp DNA gBlocks® Gene Fragments (Table 7 and Table 6) purchased from Integrated DNA Technologies (Leuven, Belgium). Each template was based on a 500 bp sequence of the *N. meningitidis NMO_1242* diagnostic target. SNPO was an exact copy of this sequence and acted as a wild-type template for positive control reactions. SNP1-6 were incomplete copies of this sequence containing single-base mismatches in close proximity to the modified LAMP primer/probe wild-type abasic site, and acted as mutant allele test templates for the modified LAMP assay. SNPA contained a single-base mismatch in close proximity to the state-of-the-art LAMP primer/probe abasic site and acted as a mutant allele test template for the state-of-the-art LAMP assay. The single-base mismatches between the gBlocks® Gene Fragment templates and their respective probes were designed to create either guanine to adenine, or cytosine to thymine, interactions (Table 7). Single-base specificity of the singleplex modified LAMP *N. meningitidis* assay was demonstrated by challenging the assay with SNP1-6 templates at 10⁵ copies (Figure 3). For comparison, the state-of-the-art LAMP assay was challenged with the SNPA template at 10⁵ copies. Positive control reactions for both assays contained *N. meningitidis* genomic DNA and the SNPO template at 10⁵ copies, with no template controls reactions performed in parallel.

| **Table 6: *N. meningitidis* 500 bp DNA gBlocks® Gene Fragments with and without SNPs.** |
|---|
| **SNPO (without SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG CCCAATTCCACATCAATACGTGAAGTCGTATGTGCAAACAGATACGTCCGCAAACCGCCCAACACAATCTCAAACAGC GACACCACCAACAAAG CCACCGACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGAAGCAACC |
| **SNP1 (with SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG |
| CCCAATTCCACATCAATACGTGAAGTCGTATGTGCAAACAGATACGTCCGCAAACCGCCCAACACAATCGCAAACAGC GACACCACCAACAAAG CCACCGACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGAAGCAACC |
| **SNP2 (with SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG CCCAATTCCACATCAATACGTGAAGTCGTATGTGCAAACAGATACGTCCGCAAACCGCCCAACACAAGCTCAAACAGC GACACCACCAACAAAG CCACCGACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGGAAGCAACC |
| **SNP3 (with SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG CCCAATTCCACATCAATACGTGAAGTCGTATGTGCAAACAGATACGTCCGCAAACCGCCCAACACACTCTCAAACAGC GACACCACCAACAAAG CCACCGACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGAAGCAACC |
| **SNP4 (with SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG CCCAATTCCACATCAATACGTGAAGTCGTATGTGCAAACAGATACGTCCGCAAACCGCCCAACACCATCTCAAACAGC GACACCACCAACAAAG CCACCGACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGAAGCAACC |
| **SNP5 (with SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG CCCAATTCCACATCAATACGTGAAGTCGTATGTGCAAACAGATACGTCCGCAAACCGCCCAACAAAATCTCAAACAGC GACACCACCAACAAAG |
| CCACCGACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGGAAGCAACC |
| **SNP6 (with SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG CCCAATTCCACATCAATACGTGAAGTCGTATGTGCAAACAGATACGTCCGCAAACCGCCCAACCCAATCTCAAACAGC GACACCACCAACAAAG CCACCGACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGGAAGCAACC |
| **SNPA (with SNP)** |
| AGCTGTAATACCACATCACCGCCAGAAAGATAAACGAAAACGCCAAATCCAACACCGAAGTCAGCGCCTGACCGGTC AAGAAATTGCGAATCTG CTCCAATTCCCGCACCCGAGCCACCGTATCACCCACTCGTCTGTGCTCGAAATAGGATAAAGGCAGGGAAAGCAGAT GCCGGAACAAACGCGCG CCCAATTCCACATCAATACGTGAAGTCGTATGTGCAAACAGATACGTCCGCAAACCGCCCAACACAATCTCAAACAGC GACACCACCAACAAAG CCACCTACACCACATCCAAAGTAGAGAATCCCCGATGTACCAGCACCTTGTCCATCACCACTTGGAAAAACAGAGGC GTAATCAGCGCAAACAG CTGCAACACCACCGACACCACCAATACTTCAAAAAACAACCGGCGGTATTTGATTACCGCCGGAATAAACCAGGTAAA GTCAAACTTTGCCAAA CTGCCCAATACCGAAGCGCGGAAGCAACC |
| Highlight, SNP mismatch. |

| **Table 7: *N. meningitidis* modified LAMP and state-of-the-art LAMP primer/probes with complementary region of each gBlocks® Gene Fragment template (SNPs highlighted).** | |
|---|---|
| **modified LAMP** | |
| **Primer/Probe Wild-Type:** | TTGA-ATTGTGTTGGGCGGTTTG ; ; ; ; ; ; ; ; ; ; ; ; ; ; ; ; ; ; ; ; ; ; ; |
| **SNP0:** | AACTCTAACACAACCCGCCAAAC |
| **SNP1:** | AACGCTAACACAACCCGCCAAAC |
| **SNP2:** | AACTCGAACACAACCCGCCAAAC |
| **SNP3:** | AACTCTCACACAACCCGCCAAAC |
| **SNP4:** | AACTCTACCACAACCCGCCAAAC |
| **SNP5:** | AACTCTAAAACAACCCGCCAAAC |
| **SNP6:** | AACTCTAACCCAACCCGCCAAAC |
| **modified LAMP** | |
| **Primer/Probe Mutant:** | TTGA-CTTGTGTTGGGCGGTTTG ;;;;;;;;;;;;;;;;;;;;;;; |
| **SNP0:** | AACTCTAACACAACCCGCCAAAC |
| **SNP2:** | AACTCGAACACAACCCGCCAAAC |
| **state-of-the-art LAMP** | |
| **Primer/Probe:** | TGTC-GTGGCTTTGTTGGTGGTGTCGCGTGCAAACAGATACGTCCG ;;;;;;;;;;;;;;;;;;;;;;;;;;;;;;;;;;;;;;;;;;;;;; |
| **SNP0:** | ACAGCCACCGAAACAACCACCACAGCGCACGTTTGTCTATGCAGGC |
| **SNPA:** | ACATCCACCGAAACAACCACCACAGCGCACGTTTGTCTATGCAGGC |
| Underline, dye labels; -, abasic site; bold, single base difference between modified LAMP Primer/Probe Wild-Type and modified LAMP Primer/Probe Mutant. | |

AS modified LAMP N. meningitidis assay single-tube detection of either wild-type or mutant allele templates

The singleplex modified LAMP *N. meningitidis* assay was modified to incorporate the modified LAMP primer/probe mutant (Table 2), a SNP2 specific mutant allele HEX fluorophore labelled modified LAMP primer/probe, also at 0.4 µM concentration, creating the AS modified LAMP *N. meningitidis* assay. This assay was separately challenged with the wild-type SNP0 template and the mutant allele SNP2 template, each at 10⁵ copies (Figure 4). Reactions were performed as per the standard singleplex modified LAMP *N. meningitidis* assay with the addition of using two fluorescence detection channels, 495-520 nm (FAM, wild-type) and 535-565 nm (HEX, mutant allele). A colour compensation file, generated as per LightCycler® 480 operator manual, was applied for correction of any channel-to-channel fluorescence crosstalk. A NTC reaction as previously described was carried out in parallel.

Multiplex modified LAMP N. meningitidis, S. pneumoniae and H. influenzae assay simultaneous multiple-target detection

The multiplex modified LAMP *N. meningitidis, S. pneumoniae* and *H. influenzae* assay was prepared as per the singleplex modified LAMP *N. meningitidis* assay, with the further addition of *S*. *pneumoniae* and *H. influenzae* modified LAMP oligonucleotides (Table 2) at the same concentration as the *N. meningitidis* oligonucleotides. The addition of molecular grade water was altered to maintain a final reaction volume of 25 µL. Simultaneous multiple-target detection using the multiplex modified LAMP *N. meningitidis, S. pneumoniae* and *H. influenzae* assay was demonstrated by challenging the assay with *N. meningitidis* NCTC 10025, *S*. *pneumoniae* DSM 20566 and *H*. *influenzae* DSM 4690 purified genomic DNA at 10² genome copies, in a single reaction (Figure 5). This reaction was performed at 65°C using fluorescence detection channels 495-520 nm (FAM), 535-565 nm (HEX) and 646-662 nm (Cy5). A colour compensation file was generated as previously described and applied for correction of any channel-to-channel fluorescence crosstalk. A NTC reaction was carried out in parallel as previously described.

### Example 1

Singleplex modified LAMP N. meningitidis assay single-target detection with comparison to state-of-the-art LAMP

Both the singleplex modified LAMP and state-of-the-art LAMP *N. meningitidis* assays successfully demonstrated single-target detection of *N. meningitidis* at 10³ genome copies (Figure 2, grey and dashed grey, respectively). However, the resulting amplification curves data highlighted significantly improved assay performance in the modified LAMP assay compared to the state-of-the-art LAMP assay. The modified LAMP assay produced time-to-positivity values of approximately 10 min compared to 20 min in the state-of-the-art LAMP assay. Additionally, the modified LAMP assay produced higher fluorescence levels compared to the state-of-the-art LAMP assay. The NTC reactions performed successfully as no amplification was observed (Figure 2, black).

### Example 2

Singleplex modified LAMP N. meningitidis assay analytical specificity, sensitivity and clinical sample testing

Complete analytical specificity was observed for the singleplex modified LAMP *N. meningitidis* assay as all *N. meningitidis* inclusivity panel reference strains were successfully detected, with no detection observed for the exclusivity panel reference strains (Table 1). The limit-of-detection (LOD) for the singleplex modified LAMP *N. meningitidis* assay was confirmed with 95% probability using Probit analysis to be 3.1 genome copies per reaction (Table 3). All IMSRL clinical samples provided, were successfully reconfirmed to be positive for the presence of respective pathogens *N. meningitidis, S. pneumoniae* or *H. influenzae,* using real-time PCR (Table 5). Compared to these PCR results, the singleplex modified LAMP *N. meningitidis* assay demonstrated 100% diagnostic sensitivity and specificity by successfully detecting all *N. meningitidis* positive clinical samples and none of the S. *pneumoniae* or *H. influenzae* positive clinical samples (Table 5). The positive controls reactions carried out in parallel were successfully detected with no detection observed in the NTC reactions.

### Example 3

Singleplex modified LAMP N. meningitidis assay SNP identification with comparison to state-of-the-art LAMP

Single-base specificity was successfully demonstrated in the singleplex modified LAMP *N*. *meningitidis* assay as only templates without SNPs, *N. meningitidis* genomic DNA (Figure 3A, grey) and the SNP0 template (Figure 3A, grey), were successfully detected. All templates incorporating SNPs, SNP1-6, were not detected with the modified LAMP assay (Figure 3A, dashed black). The singleplex state-of-the-art LAMP *N. meningitidis* assay successfully detected templates without SNPs, *N. meningitidis* genomic DNA (Figure 3B, grey) and the SNP0 template (Figure 3B, grey). However, the state-of-the-art LAMP assay did not demonstrate single-base specificity as it also detected the SNP incorporating template, SNPA (Figure 3B, dashed grey). The no template control reactions performed successfully in both assays as no signal was observed (Figure 3, black).

### Example 4

Allele-specific (AS) modified LAMP N. meningitidis assay single-tube detection of either wild-type or mutant allele templates

The AS modified LAMP *N. meningitidis* assay, incorporating FAM labelled wild-type modified LAMP primer/probe and HEX labelled mutant SNP2 specific modified LAMP primer/probe, successfully demonstrated differential detection of wild-type or mutant allele templates at 10⁵ copies, in single-tube reactions (Figure 4). The SNP0 template, acting as the wild-type template, was successfully detected by the wild-type specific FAM labelled modified LAMP primer/probe in the FAM detection channel (Figure 4A, grey), with no detection of this template observed in the HEX detection channel (Figure 4B, grey). The SNP2 template, acting as the mutant allele template, was successfully detected by the mutant allele SNP2 specific HEX labelled modified LAMP primer/probe in the HEX detection channel (Figure 4B, dashed grey), with no detection of this template observed in the FAM detection channel (Figure 4A, dashed grey). The NTC reaction performed successfully as no signal was observed (Figure 4, black)

### Example 5

Multiplex modified LAMP N. meningitidis, S. pneumoniae and H. influenzae assay simultaneous multiple-target detection

Simultaneous multiple-target detection of all three bacterial templates at 10² genome copies, in a single reaction, was successfully demonstrated using the multiplex modified LAMP *N. meningitidis, S. pneumoniae* and *H. influenzae* assay (Figure 5, dashed black). Each target was detected in the appropriate respective fluorescence detection channel of the LightCycler® 480, *N. meningitidis* FAM (Figure 5A), *S*. *pneumoniae* HEX (Figure 5B) and *H. influenzae* Cy5 (Figure 5C). The NTC reaction performed successfully as no amplification was observed (Figure 5, black).

### Example 6

Singleplex modified LAMP C. albicans assay single-target detection

Single-target detection of C. albicans bacterial template at 10^5 genome copies was successfully demonstrated using the singleplex C. albicans modified LAMP assay (Figure 6, grey). The NTC reaction performed successfully as no amplification was observed (Figure 6, black).

### Discussion

Loop-mediated isothermal amplification (LAMP) provides rapid, robust, sensitive and specific, user-friendly, nucleic acid amplification technology for POC infectious disease diagnostics in low-resourced disease burdened areas. However, multiplex pathogen detection and SNP identification using LAMP is difficult to achieve. Nucleic acid diagnostics requires multiplex detection capabilities to facilitate simultaneous multiple-pathogen detection, reduced analysis time, sample conservation and incorporation of internal control validation. SNP identification capabilities in nucleic acid diagnostics enables effective differentiation of closely related pathogens, identification of point-mutations associated with antimicrobial resistance and more effective disease epidemiological surveillance/control. The present invention provides technology for singleplex or multiplex pathogen detection with SNP identification. The invention demonstrates single-target detection and SNP identification using a singleplex modified LAMP assay, with comparison to the previously reported state-of-the-art LAMP technology and evaluation in terms of analytical specificity, sensitivity and clinical application. Modified versions of this assay were subsequently used to demonstrate single-tube wild-type and mutant allele differentiation, and simultaneous multiple-pathogen detection.

The singleplex modified LAMP *N. meningitidis* assay demonstrated earlier detection and increased fluorescence production compared to state-of-the-art LAMP (Figure 2), highlighting improved assay performance. This result is possibly due to enhanced reaction kinetics resulting from reduced cleavage enzyme present in modified LAMP compared to state-of-the-art LAMP, and increased cleavage activity of Endonuclease IV in modified LAMP, compared to *Tth* Endonuclease IV in state-of-the-art LAMP. Also, perhaps the 5'-end inner primer state-of-the-art LAMP modifications effect loop structure formation compared to the unmodified inner primers in modified LAMP. The singleplex modified LAMP *N. meningitidis* assay was 100% analytically specific, as only the inclusivity panel reference strains were detected during specificity testing (Table 1). The LOD of the singleplex modified LAMP *N. meningitidis* assay was established to be 3.1 genome copies per reaction using Probit analysis (Table 3). Typical LODs for previously reported *N. meningitidis* LAMP assays range from 6-10 genome copies per reaction. Small scale clinical sample testing of the singleplex modified LAMP *N. meningitidis* assay, using DNA from *N. meningitidis, S. pneumoniae* and *H. influenzae* positive blood and CSF samples, demonstrated 100% diagnostic sensitivity and specificity as only the *N. meningitidis* samples were detected (Table 5).

The invention demonstrates that the previously reported state-of-the-art LAMP technology does not enable single-base specificity as it amplified templates with and without SNPs located in the state-of-the-art LAMP primer/probe target region (Figure 3B). Presence of a 5'-end SNP in the state-of-the-art LAMP primer/probe target region may inhibit binding during loop formation, preventing abasic site dsDNA formation and inhibiting cleavage, which should subsequently enable SNP identification. Without being bound by theory, it is hypothesised that during *Bst* polymerisation dissociating the loop structure, extension over the state-of-the-art LAMP primer/probe abasic site occurs, producing dsDNA which enables cleavage and fluorescence production regardless of the presence of a SNP.

The present invention successfully demonstrates single-base specificity as none of the SNP containing templates tested, SNP1-6 (Table 6), were detected (Figure 3A). Without being bound by theory, this single-base specificity in modified LAMP may be due to a combination of reaction temperature with the presence of a SNP inhibiting efficient modified LAMP primer/probe target hybridisation, which prevents cleavage of the abasic site. The demonstration of SNP identification in this study (Figure 3A) further indicates that nucleotide mismatches located within a specific 6 base region of the modified LAMP primer/probe (Table 7) prevents detection. SNPs located directly outside this 6 base range on the modified LAMP primer/probe were also tested but identified as positive with the LightCycler® 480. However, the resulting amplification curves for these templates indicated low level detection which was easily differentiable from the positive control reactions used. Based on these results, the present invention demonstrates flexible assay design for obtaining SNP identification as targeted nucleotide mismatches can be located anywhere within this 6 base region (Table 7). There is currently no other real-time multiplex isothermal nucleic acid amplification method that offers this type of flexible SNP identification. Further to this, utilising the loop primer for SNP identification enables even greater design flexibility, as once the core inner and outer primer sequences are confirmed, different loop primers can be designed to target various sequences, and SNPs, located in the loop section. SNP identification was demonstrated using synthetic 500 bp DNA gBlocks® Gene Fragments instead of genomic DNA. However, the resulting modified LAMP and state-of-the-art LAMP amplification curves produced with these templates were very similar to resulting *N. meningitidis* genomic DNA amplification curves (Figure 3).

Modifications were made to the singleplex modified LAMP assay, including addition of a mutant modified LAMP primer/probe (Table 2) to create the allele-specific (AS) modified LAMP assay, and addition of *S*. *pneumoniae* and *H. influenzae* oligonucleotides (Table 2) to create the multiplex modified LAMP assay. The AS modified LAMP assay successfully demonstrated a single-tube reaction that can detect either wild-type or mutant allele templates (Figure 4). Other LAMP technologies reporting SNP genotyping capabilities require separate reactions to independently detect wild-type or mutant allele templates, increasing reagent and sample specimen costs. The multiplex modified LAMP assay successfully demonstrated the simultaneous detection of *N*. *meningitidis, S. pneumoniae* and *H. influenzae* (Figure 5) in a single reaction. This result highlights the robustness of modified LAMP in terms of tolerance to co-amplification inhibition which is necessary for multiplex nucleic acid diagnostics in the occurrence of pathogen co-infection. The demonstration of multiplex modified LAMP detection also highlights the potential of this technology for incorporation of internal amplification controls, as with state-of-the-art LAMP. Multiplex modified LAMP was performed at the lower temperature of 65°C, compared to 67°C in singleplex modified LAMP, as this produced optimal detection of all three bacterial targets while maintaining efficient SNP identification capabilities. The combination of AS modified LAMP and multiplex modified LAMP capabilities highlights the potential for multiplex detection of closely positioned SNPs using a single core LAMP primer set. Previous PCR-based SNP genotyping technologies have been reported for the detection of SNPs clustered together in close proximity. Based on the multiplex capabilities of modified LAMP, the addition of further modified LAMP primer/probes to the AS modified LAMP assay, each targeting different SNPs and labelled with alternative fluorophores, could enable simultaneous detection of multiple closely located SNPs.

Various LAMP technologies with SNP and point-mutation genotyping capabilities have been reported. However, these methodologies have limitations compared to the present invention. Most reported methods employ allele-specific forward and reverse inner primers with overlapping 5'-end mismatches to a specific SNP, preventing hybridisation and amplification, and thus enabling differentiation between templates with and without this SNP. This strategy, however, has restrictive assay design as the entire LAMP primer set is based around a single nucleotide location. Also, this approach cannot guarantee complete SNP template amplification suppression, often leading to delayed SNP template detection and the occurrence of false positives or non-specific amplification. Monitoring this method is generally performed using intercalating dyes, which can inhibit LAMP reactions, or real-time turbidimetry. Both monitoring approaches, however, do not facilitate multiplex detection and require extensive optimisation to avoid non-specific amplification. It has been reported that SNP LAMP techniques using allele-specific oligonucleotide hybridisation (LAMP-ASO) and annealing selectivity of allele-specific inner primers with 3'-end or 5'-end mismatches (3'AS-LAMP and 5'AS-LAMP). However, LAMP-ASO is not a single-tube system requiring contamination prone post-amplification processing, and the AS-LAMP methods are prone to false-positive results. Also reported is the use of gold nanoparticles to achieve SNP LAMP. This method, however, is non-isothermal as template denaturing at 95°C is required, with laborious contamination prone post-amplification processing. Also reported is use of gold nanoparticles to achieve SNP LAMP, however, this method again requires post-amplification analysis.

The present invention demonstrates that the inner primers are not suitable for achieving LAMP SNP identification with the state-of-the-art LAMP primer/probe modifications (Figure 3B). Also, considering the outer primers do not target the unique double-looped LAMP template structure, the loop primers were chosen for development of modified LAMP technology. A small number of LAMP technologies have reported real-time detection and SNP identification capabilities through utilisation of the loop primers. FLOS-LAMP demonstrates real-time LAMP detection using self-quenching and de-quenching fluorogenic loop probes. However, this technology has not demonstrated multiplex target detection, does not enable SNP identification, has a relatively poor LOD of 500 genome copies, and possesses restrictive design limitations requiring guanine or cytosine flanked thymine residues for probe design. PNA-LNA mediated LAMP has demonstrated SNP detection capabilities via an amplification blocking peptide nucleic acid (PNA) clamping probe that targets the loop regions in LAMP. However, this method does not enable multiplex detection and requires post-amplification PCR or gel electrophoresis analysis. LAMP-FLP incorporates a fluorescently labelled loop primer (FLP), and quencher probe (QP), enabling SNP detection by measuring resulting peak temperatures of fluorescence resonance energy transfer (FRET). This method, however, requires increased oligonucleotide primer compared to standard LAMP, and is not fully isothermal as generation of a post-amplification annealing curve from 95°C to 35°C is required. It is also reported that a SNP LAMP technique using a similar principle to the SNP overlap allele-specific inner primer methods.

This method, however, utilises overlap between the loop and inner primer, and is subject to the same previously mentioned limitations of restrictive assay design and monitoring using intercalating dyes or turbidity.

The present invention incorporates all of the previously reported properties of state-of-the-art LAMP technology, in terms of assay specificity, sensitivity and improved multiplex target detection over competing methods. However, the present invention further improves on state-of-the-art LAMP in terms of reaction time-to-positivity and fluorescence production, as well as now incorporating flexible SNP identification capabilities. Additionally, the present invention requires one fifteenth of the cleavage enzyme concentration, and half of the oligonucleotide probe concentration, required by state-of-the-art LAMP. The modified LAMP primer/probe also uses alternate fluorophore and quencher positioning to state-of-the-art LAMP, this has no impact on assay performance but further reduces assay cost in terms of oligonucleotide synthesis. Further reduced assay costs can be achieved by lowering the modified LAMP primer and probe concentrations by half, maintaining comparable detection times and fluorescence production with single-digit genome copy LOD. the present invention is the first report of a single-tube, real-time, multiplex LAMP method with SNP identification capabilities, providing state-of-the-art transferable isothermal nucleic acid amplification technology for POC infectious disease diagnostics.

## Claims

1. A set of primers for loop-mediated isothermal amplification (LAMP) comprising
(a) a forward outer primer;
(b) a reverse outer primer;
(c) a forward inner primer;
(d) a reverse inner primer,
(e) a forward loop primer; and
(f) a reverse loop primer;
wherein each primer is capable of binding to a target nucleic acid having complementary first and second nucleic acid strands,
wherein the first nucleic acid strand has first, second, third, fourth, fifth and sixth regions; and
wherein the second nucleic acid strand has first, second, third, fourth, fifth and sixth regions;
wherein the fourth, fifth and sixth regions of the first nucleic acid strand are complimentary to the third, second, and first regions of the second nucleic acid strand; and
wherein the fourth, fifth and sixth regions of the second nucleic acid strand are complimentary to the third, second, and first regions of the first nucleic acid strand; and
wherein
(a) the forward outer primer is complementary to the first region of the first nucleic acid strand;
(b) the reverse outer primer is complementary to the first region of the second nucleic acid strand;
(c) the forward inner primer has first and second parts, wherein:
(i) the first part is complementary to the second region of the first nucleic acid strand; and
(ii) the second part is complementary to the fourth region of the second nucleic acid strand; and
(d) the reverse inner primer has first and second parts, wherein:
(i) the first part is complementary to the second region of the second nucleic acid strand; and
(ii) the second part is complementary to the fourth region of the first nucleic acid strand;
(e) the forward loop primer is complementary to a region between the fourth and fifth regions of the second nucleic acid strand;
(f) the reverse loop primer is complementary to a region between the fourth and fifth regions of the second nucleic acid strand;
wherein at least one of the (e) forward loop and (f) reverse loop primers comprises a reporter.

2. A set of primers according to Claim 1, wherein the (e) forward loop primer comprises the reporter.

3. A set of primers according to Claim 1, wherein the (f) reverse loop primer comprises the reporter.

4. A set of primers according to any one of Claims 1-3, wherein the primer further comprises an endonuclease cleavage site.

5. A set of primers according to Claim 4, wherein the cleavage site is an apurinic/apyrimidinic site (abasic site).

6. A set of primers according to any one of Claims 1-5, wherein the reporter comprises first and second dyes.

7. A set of primers according to Claim 4, wherein the first and second dyes are located at or adjacent opposing ends of the cleavage site.

8. A set of primers according to any one of Claims 1-7, wherein reporter comprises a dye and a quencher.

9. A set of primers according to Claim 8, wherein the dye is selected from 3',6'-dihydroxy-1-oxospiro[2-benzofuran-3,9'-xanthene]-5-carboxylic acid (6-Carboxyfluorescein; 6-FAM); 6-Carboxy-2',7'-dichlorofluorescein diacetate N-succinimidyl ester (6-hexachlorofluorescein; 6-HEX); and 1-{6-[(2,5-Dioxo-1-pyrrolidinyl)oxy]-6-oxohexyl}-2-[(1E,3E,5E)-5-(1-{6-[(2,5-dioxo-1-pyrrolidinyl)oxy]-6-oxohexyl}-3,3-dimethyl-5-sulfo-1,3-dihydro-2H-indol-2-ylidene)-1,3-pentadien-1-yl]-3,3-dimethyl-3H-indolium-5-sulfonate (cyanine; Cy5).

10. A set of primers according to Claim 8 or 9, wherein the quencher is a dark quencher.

11. A method for identifying a target nucleic acid, the method comprising:
(a) providing a sample;
(b) providing a set of primers according to any one of Claims 1-10;
(c) performing isothermal amplification; and
(d) identifying the nucleic acid target in the sample.

12. A method for identifying a nucleic acid modification or substitution of a target nucleic acid, the method comprising:
(a) providing a sample;
(b) providing a set of primers according to any one of Claims 1-10;
(c) performing isothermal amplification; and
(d) identifying the nucleic acid modification or substitution of the target nucleic acid in the sample.

13. A method according to Claim 11 or 12, wherein the isothermal amplification is performed in the presence of a Bacillus stearothermophilus deoxyribonucleic acid polymerase enzyme and an Endonuclease IV enzyme.

14. A method according to any one of Claims 11-13, wherein the isothermal amplification is performed at a temperature of 65-67°C.

15. A method according to any one of Claims 11-14, wherein the isothermal amplification is performed for 60 minutes.


SEQUENCE LISTING

<110>  NATIONAL UNIVERSITY OF IRELAND, GALWAY

<120>  Primers for isothermal amplification

<130>  P128920EP00

<160>  59

<170>  PatentIn version 3.5

<210>  1
<211>  46
<212>  DNA
<213>  Unknown

<220>
<223>  Synthetic Primer Sequence

<400>  1
tgtcggtggc tttgttggtg gtgtcgcgtg caaacagata cgtccg                    46


<210>  2
<211>  42
<212>  DNA
<213>  Unknown

<220>
<223>  Synthetic Primer Sequence

<400>  2
ccgatgtacc agcaccttgt ccgtttgcgc tgattacgcc tc                        42


<210>  3
<211>  22
<212>  DNA
<213>  Unknown

<220>
<223>  Synthetic Primer Sequence

<400>  3
cccaattcca catcaatacg tg                                              22


<210>  4
<211>  22
<212>  DNA
<213>  Unknown

<220>
<223>  Synthetic Primer Sequence

<400>  4
ttgaattgtg ttgggcggtt tg                                              22


<210>  5
<211>  22
<212>  DNA

<213>  Unknown

<220>
<223>  Synthetic Primer Sequence

<400>  5
ttgacttgtg ttgggcggtt tg                                                22


<210>  6
<211>  22
<212>  DNA
<213>  Unknown

<220>
<223>  Synthetic Primer Sequence

<400>  6
caccacttgg aaaaacagag gc                                                22


<210>  7
<211>  47
<212>  DNA
<213>  Unknown

<220>
<223>  Synthetic Primer Sequence

<400>  7
tggaaaatgc tctggctttt gaagtgacct acaccaatat cctcgct                     47


<210>  8
<211>  47
<212>  DNA
<213>  Unknown

<220>
<223>  Synthetic Primer Sequence

<400>  8
tctgtctggt agacagaatg acggatcttt gagaatcaga tgctgga                     47


<210>  9
<211>  18
<212>  DNA
<213>  Unknown

<220>
<223>  Synthetic Primer Sequence

<400>  9
tccgtcaacg aggcacaa                                                     18


<210>  10
<211>  18
<212>  DNA
<213>  Unknown

<220>

<223>  Synthetic Primer Sequence

<400>  10
agcaaactca ccaagcgc                                                    18


<210>  11
<211>  24
<212>  DNA
<213>  Unknown

<220>
<223>  Synthetic Primer Sequence

<400>  11
tgatgaaaca gacaagctga ttct                                             24


<210>  12
<211>  27
<212>  DNA
<213>  Unknown

<220>
<223>  Synthetic Primer Sequence

<400>  12
actccatgcg caatgatggt ataatcc                                          27


<210>  13
<211>  49
<212>  DNA
<213>  Unknown

<220>
<223>  Synthetic Primer Sequence

<400>  13
tgccgctgct tcacgtaaat tatttggtgc ttattcctat cgtggtacg                  49


<210>  14
<211>  42
<212>  DNA
<213>  Unknown

<220>
<223>  Synthetic Primer Sequence

<400>  14
cttggttgct ctcaatggca aggcacgcca gttaaaatcc ct                         42


<210>  15
<211>  18
<212>  DNA
<213>  Unknown

<220>
<223>  Synthetic Primer Sequence

<400>  15

ggctggagca ttcgcatt                                                    18


<210>  16
<211>  20
<212>  DNA
<213>  Unknown

<220>
<223>  Synthetic Primer Sequence

<400>  16
ttctcctgaa attcgggcaa                                                  20


<210>  17
<211>  20
<212>  DNA
<213>  Unknown

<220>
<223>  Synthetic Primer Sequence

<400>  17
aacatattgt ccgtagtgcg                                                  20


<210>  18
<211>  25
<212>  DNA
<213>  Unknown

<220>
<223>  Synthetic Primer Sequence

<400>  18
ttgtatcgag cagctaaatc aggga                                            25


<210>  19
<211>  45
<212>  DNA
<213>  Unknown

<220>
<223>  Synthetic Primer Sequence

<400>  19
tgtcgtggct ttgttggtgg tgtcgcgtgc aaacagatac gtccg                      45


<210>  20
<211>  46
<212>  DNA
<213>  Unknown

<220>
<223>  Synthetic Primer Sequence

<400>  20
tgtcggtggc tttgttggtg gtgtcgcgtg caaacagata cgtccg                     46

<210>  21
<211>  42
<212>  DNA
<213>  Unknown

<220>
<223>  Synthetic Primer Sequence

<400>  21
ccgatgtacc agcaccttgt ccgtttgcgc tgattacgcc tc                          42


<210>  22
<211>  22
<212>  DNA
<213>  Unknown

<220>
<223>  Synthetic Primer Sequence

<400>  22
cccaattcca catcaatacg tg                                                22


<210>  23
<211>  18
<212>  DNA
<213>  Unknown

<220>
<223>  Synthetic Primer Sequence

<400>  23
gtggtgtcgg tggtgttg                                                     18


<210>  24
<211>  23
<212>  DNA
<213>  Unknown

<220>
<223>  Synthetic Primer Sequence

<400>  24
ttgagattgt gttgggcggt ttg                                               23


<210>  25
<211>  22
<212>  DNA
<213>  Unknown

<220>
<223>  Synthetic Primer Sequence

<400>  25
caccacttgg aaaaacagag gc                                                22


<210>  26
<211>  23
<212>  DNA

<213>  Unknown

<220>
<223>  Synthetic Primer Sequence

<400>  26
cgacatgttc gaacgtaatc tcc                                              23


<210>  27
<211>  24
<212>  DNA
<213>  Unknown

<220>
<223>  Synthetic Primer Sequence

<400>  27
tatcgggcaa agccaaatgc gaag                                             24


<210>  28
<211>  17
<212>  DNA
<213>  Unknown

<220>
<223>  Synthetic Primer Sequence

<400>  28
atttcggtgg cgcgttt                                                     17


<210>  29
<211>  21
<212>  DNA
<213>  Unknown

<220>
<223>  Synthetic Primer Sequence

<400>  29
ctcgtaagcg taaactcctt g                                                21


<210>  30
<211>  25
<212>  DNA
<213>  Unknown

<220>
<223>  Synthetic Primer Sequence

<400>  30
acgcatgaaa tccatcggat cagtt                                            25


<210>  31
<211>  20
<212>  DNA
<213>  Unknown

<220>

<223>  Synthetic Primer Sequence

<400>  31
catactcaag acgctgagga                                                    20


<210>  32
<211>  22
<212>  DNA
<213>  Unknown

<220>
<223>  Synthetic Primer Sequence

<400>  32
ggtacgcacy acggacaata tg                                                 22


<210>  33
<211>  24
<212>  DNA
<213>  Unknown

<220>
<223>  Synthetic Primer Sequence

<400>  33
agctcttggt tgctctcaat ggca                                               24


<210>  34
<211>  23
<212>  DNA
<213>  Unknown

<220>
<223>  Synthetic Primer Sequence

<400>  34
cctgatttag cygctcgata aca                                                23


<210>  35
<211>  500
<212>  DNA
<213>  Neisseria meningitidis

<400>  35
agctgtaata ccacatcacc gccagaaaga taaacgaaaa cgccaaatcc aacaccgaag        60

tcagcgcctg accggtcaag aaattgcgaa tctgctccaa ttcccgcacc cgagccaccg       120

tatcacccac tcgtctgtgc tcgaaatagg ataaaggcag ggaaagcaga tgccggaaca       180

aacgcgcgcc caattccaca tcaatacgtg aagtcgtatg tgcaaacaga tacgtccgca       240

aaccgcccaa cacaatctca aacagcgaca ccaccaacaa agccaccgac accacatcca       300

aagtagagaa tccccgatgt accagcacct tgtccatcac cacttggaaa aacagaggcg       360

taatcagcgc aaacagctgc aacaccaccg acaccaccaa tacttcaaaa aacaaccggc       420

ggtatttgat taccgccgga ataaaccagg taaagtcaaa ctttgccaaa ctgcccaata       480

ccgaagcgcg ggaagcaacc 500

<210> 36
<211> 500
<212> DNA
<213> Neisseria meningitidis

<400> 36
agctgtaata ccacatcacc gccagaaaga taaacgaaaa cgccaaatcc aacaccgaag 60

tcagcgcctg accggtcaag aaattgcgaa tctgctccaa ttcccgcacc cgagccaccg 120

tatcacccac tcgtctgtgc tcgaaatagg ataaaggcag ggaaagcaga tgccggaaca 180

aacgcgcgcc caattccaca tcaatacgtg aagtcgtatg tgcaaacaga tacgtccgca 240

aaccgcccaa cacaatcgca aacagcgaca ccaccaacaa agccaccgac accacatcca 300

aagtagagaa tccccgatgt accagcacct tgtccatcac cacttggaaa aacagaggcg 360

taatcagcgc aaacagctgc aacaccaccg acaccaccaa tacttcaaaa aacaaccggc 420

ggtatttgat taccgccgga ataaaccagg taaagtcaaa ctttgccaaa ctgcccaata 480

ccgaagcgcg ggaagcaacc 500

<210> 37
<211> 500
<212> DNA
<213> Neisseria meningitidis

<400> 37
agctgtaata ccacatcacc gccagaaaga taaacgaaaa cgccaaatcc aacaccgaag 60

tcagcgcctg accggtcaag aaattgcgaa tctgctccaa ttcccgcacc cgagccaccg 120

tatcacccac tcgtctgtgc tcgaaatagg ataaaggcag ggaaagcaga tgccggaaca 180

aacgcgcgcc caattccaca tcaatacgtg aagtcgtatg tgcaaacaga tacgtccgca 240

aaccgcccaa cacaagctca aacagcgaca ccaccaacaa agccaccgac accacatcca 300

aagtagagaa tccccgatgt accagcacct tgtccatcac cacttggaaa aacagaggcg 360

taatcagcgc aaacagctgc aacaccaccg acaccaccaa tacttcaaaa aacaaccggc 420

ggtatttgat taccgccgga ataaaccagg taaagtcaaa ctttgccaaa ctgcccaata 480

ccgaagcgcg ggaagcaacc 500

<210> 38
<211> 500
<212> DNA
<213> Neisseria meningitidis

<400> 38
agctgtaata ccacatcacc gccagaaaga taaacgaaaa cgccaaatcc aacaccgaag 60

tcagcgcctg accggtcaag aaattgcgaa tctgctccaa ttcccgcacc cgagccaccg 120

tatcacccac tcgtctgtgc tcgaaatagg ataaaggcag ggaaagcaga tgccggaaca 180

aacgcgcgcc caattccaca tcaatacgtg aagtcgtatg tgcaaacaga tacgtccgca 240

aaccgcccaa cacactctca aacagcgaca ccaccaacaa agccaccgac accacatcca 300

aagtagagaa tccccgatgt accagcacct tgtccatcac cacttggaaa aacagaggcg 360

taatcagcgc aaacagctgc aacaccaccg acaccaccaa tacttcaaaa aacaaccggc 420

ggtatttgat taccgccgga ataaaccagg taaagtcaaa ctttgccaaa ctgcccaata 480

ccgaagcgcg ggaagcaacc 500

<210> 39
<211> 500
<212> DNA
<213> Neisseria meningitidis

<400> 39
agctgtaata ccacatcacc gccagaaaga taaacgaaaa cgccaaatcc aacaccgaag 60

tcagcgcctg accggtcaag aaattgcgaa tctgctccaa ttcccgcacc cgagccaccg 120

tatcacccac tcgtctgtgc tcgaaatagg ataaaggcag ggaaagcaga tgccggaaca 180

aacgcgcgcc caattccaca tcaatacgtg aagtcgtatg tgcaaacaga tacgtccgca 240

aaccgcccaa caccatctca aacagcgaca ccaccaacaa agccaccgac accacatcca 300

aagtagagaa tccccgatgt accagcacct tgtccatcac cacttggaaa aacagaggcg 360

taatcagcgc aaacagctgc aacaccaccg acaccaccaa tacttcaaaa aacaaccggc 420

ggtatttgat taccgccgga ataaaccagg taaagtcaaa ctttgccaaa ctgcccaata 480

ccgaagcgcg ggaagcaacc 500

<210> 40
<211> 500
<212> DNA
<213> Neisseria meningitidis

<400> 40
agctgtaata ccacatcacc gccagaaaga taaacgaaaa cgccaaatcc aacaccgaag 60

tcagcgcctg accggtcaag aaattgcgaa tctgctccaa ttcccgcacc cgagccaccg 120

tatcacccac tcgtctgtgc tcgaaatagg ataaaggcag ggaaagcaga tgccggaaca 180

aacgcgcgcc caattccaca tcaatacgtg aagtcgtatg tgcaaacaga tacgtccgca 240

aaccgcccaa caaaatctca aacagcgaca ccaccaacaa agccaccgac accacatcca 300

aagtagagaa tccccgatgt accagcacct tgtccatcac cacttggaaa aacagaggcg 360

taatcagcgc aaacagctgc aacaccaccg acaccaccaa tacttcaaaa aacaaccggc 420

ggtatttgat taccgccgga ataaaccagg taaagtcaaa ctttgccaaa ctgcccaata 480

ccgaagcgcg ggaagcaacc 500

<210> 41
<211> 500
<212> DNA
<213> Neisseria meningitidis

<400> 41
agctgtaata ccacatcacc gccagaaaga taaacgaaaa cgccaaatcc aacaccgaag 60

tcagcgcctg accggtcaag aaattgcgaa tctgctccaa ttcccgcacc cgagccaccg 120

tatcacccac tcgtctgtgc tcgaaatagg ataaaggcag ggaaagcaga tgccggaaca 180

aacgcgcgcc caattccaca tcaatacgtg aagtcgtatg tgcaaacaga tacgtccgca 240

aaccgcccaa cccaatctca aacagcgaca ccaccaacaa agccaccgac accacatcca 300

aagtagagaa tccccgatgt accagcacct tgtccatcac cacttggaaa aacagaggcg 360

taatcagcgc aaacagctgc aacaccaccg acaccaccaa tacttcaaaa aacaaccggc 420

ggtatttgat taccgccgga ataaaccagg taaagtcaaa ctttgccaaa ctgcccaata 480

ccgaagcgcg ggaagcaacc 500

<210> 42
<211> 500
<212> DNA
<213> Neisseria meningitidis

<400> 42
agctgtaata ccacatcacc gccagaaaga taaacgaaaa cgccaaatcc aacaccgaag 60

tcagcgcctg accggtcaag aaattgcgaa tctgctccaa ttcccgcacc cgagccaccg 120

tatcacccac tcgtctgtgc tcgaaatagg ataaaggcag ggaaagcaga tgccggaaca 180

aacgcgcgcc caattccaca tcaatacgtg aagtcgtatg tgcaaacaga tacgtccgca 240

aaccgcccaa cacaatctca aacagcgaca ccaccaacaa agccacctac accacatcca 300

aagtagagaa tccccgatgt accagcacct tgtccatcac cacttggaaa aacagaggcg 360

taatcagcgc aaacagctgc aacaccaccg acaccaccaa tacttcaaaa aacaaccggc 420

ggtatttgat taccgccgga ataaaccagg taaagtcaaa ctttgccaaa ctgcccaata 480

ccgaagcgcg ggaagcaacc 500

<210> 43
<211> 23
<212> DNA
<213> Unknown

<220>
<223> Synthetic Primer Sequence

<400> 43
aactctaaca caacccgcca aac 23

<210>  44
<211>  23
<212>  DNA
<213>  Unknown

<220>
<223>  Synthetic Primer Sequence

<400>  44
aacgctaaca caacccgcca aac                                              23


<210>  45
<211>  23
<212>  DNA
<213>  Unknown

<220>
<223>  Synthetic Primer Sequence

<400>  45
aactcgaaca caacccgcca aac                                              23


<210>  46
<211>  23
<212>  DNA
<213>  Unknown

<220>
<223>  Synthetic Primer Sequence

<400>  46
aactctcaca caacccgcca aac                                              23


<210>  47
<211>  23
<212>  DNA
<213>  Unknown

<220>
<223>  Synthetic Primer Sequence

<400>  47
aactctacca caacccgcca aac                                              23


<210>  48
<211>  23
<212>  DNA
<213>  Unknown

<220>
<223>  Synthetic Primer Sequence

<400>  48
aactctaaaa caacccgcca aac                                              23


<210>  49

<211>  23
<212>  DNA
<213>  Unknown

<220>
<223>  Synthetic Primer Sequence

<400>  49
aactctaacc caacccgcca aac                                              23


<210>  50
<211>  23
<212>  DNA
<213>  Unknown

<220>
<223>  Synthetic Primer Sequence

<400>  50
aactctaaca caacccgcca aac                                              23


<210>  51
<211>  23
<212>  DNA
<213>  Unknown

<220>
<223>  Synthetic Primer Sequence

<400>  51
aactcgaaca caacccgcca aac                                              23


<210>  52
<211>  46
<212>  DNA
<213>  Unknown

<220>
<223>  Synthetic Primer Sequence

<400>  52
acagccaccg aaacaaccac cacagcgcac gtttgtctat gcaggc                     46


<210>  53
<211>  46
<212>  DNA
<213>  Unknown

<220>
<223>  Synthetic Primer Sequence

<400>  53
acatccaccg aaacaaccac cacagcgcac gtttgtctat gcaggc                     46


<210>  54
<211>  21
<212>  DNA
<213>  Unknown

<220>
<223>  Synthetic primer

<400>  54
caccagaatt taggtgccat g                                                21


<210>  55
<211>  22
<212>  DNA
<213>  Unknown

<220>
<223>  Synthetic primer

<400>  55
gcggagttga ttgttttggt tg                                               22


<210>  56
<211>  49
<212>  DNA
<213>  Unknown

<220>
<223>  Synthetic primer

<400>  56
tgatgtggtt gtggttgtgg ttgcgatatc aacttttata cacccatgt                  49


<210>  57
<211>  50
<212>  DNA
<213>  Unknown

<220>
<223>  Synthetic primer

<400>  57
acagcatcaa cctgaattac aaaacactac tgatgttgta gcatttgctg                 50


<210>  58
<211>  21
<212>  DNA
<213>  Unknown

<220>
<223>  Synthetic primer

<400>  58
ttcactgtac tggaagctcg t                                                21


<210>  59
<211>  26
<212>  DNA
<213>  Unknown

<220>
<223>  Synthetic primer

<400>  59
ccaacaaatt aaacacattc aacagc                                          26